# EUROPEAN PATENT SPECIFICATION

(11) **EP 1 042 006 B1**
(45) Date of publication and mention of the grant of the patent: **22.08.2001**
(21) Application number: 98958391.9
(22) Date of filing: 01.12.1998
(51) Int. Cl.: A61L 2/08

(54) **A METHOD FOR DISINFECTING LIQUIDS AND GASES AND DEVICES FOR USE THEREOF**
VERFAHREN ZUR DESINFEKTION VON FLÜSSIGKEITEN UND GASEN UND GERÄTE ZU DESSEN ANWENDUNG
PROCEDE DE DESINFECTION DE LIQUIDES ET DE GAZ ET DISPOSITIFS UTILISABLES A CETTE FIN

(30) Priority: 01.12.1997 IL 12238897
(43) Date of publication of application: 11.10.2000
(73) Proprietor: Tribelski, Zamir, 95744 Jerusalem (IL)
(72) Inventor: Tribelski, Zamir, 95744 Jerusalem (IL)
(74) Representative: Benedum, Ulrich Max, Dr.
(86) International application number: PCT/IL98/00589
(87) International publication number: WO 99/27970

(56) References cited:
- WO-A-92/18170
- WO-A-95/13098
- WO-A-96/04935
- US-A- 5 262 066
- PATENT ABSTRACTS OF JAPAN vol. 097, no. 001, 31 January 1997 & JP 08 238476 A (TOSHIBA CORP), 17 September 1996
- PATENT ABSTRACTS OF JAPAN vol. 016, no. 202 (C-0940), 14 May 1992 & JP 04 033659 A (SANBETSUKU KK;OTHERS: 01), 5 February 1992
- PATENT ABSTRACTS OF JAPAN vol. 010, no. 296 (C-377), 8 October 1986 & JP 61 111189 A (SUMITOMO JUKIKAI ENVIROTEC KK), 29 May 1986
- PATENT ABSTRACTS OF JAPAN vol. 013, no. 137 (C-582), 5 April 1989 & JP 63 302940 A (SUMITOMO ELECTRIC IND LTD), 9 December 1988

## Description

### FIELD OF THE INVENTION

The present invention relates to a novel method for disinfecting liquids and gases and to devices using this method. More specifically, the present invention relates to a methods for disinfecting liquids and gasses by light which is radiated into said liquids and gasses by optical fibers. Said light may be ultra violet light (UVA, UVB, UVC) which is especially useful for killing bacteria or microscopic noxious microorganism (such as those passing through filtration units). The light may be also in the visible region of the spectrum, which is especially useful for disturbing the breeding cycle of cockroaches (such as those living in sewage networks or other close spaces). Or in any other spectral range suitable for killing noxious microorganisms.

### BACKGROUND TO THE INVENTION

Radiation is known to effect many species population factors in natural, industrial, and domestic ecological systems. The term "radiation" in the context of the present invention includes all the spectral ranges including the visible spectrum i.e. illumination. Radiation of one frequency may effect an increase in the population of one species while simultaneously causing the inactivation or elimination of other species (disinfecting).

For the purposes of the present invention, the term "disinfecting" relates to reducing the population of any noxious species. (e.g. selective inactivation and/or destruction of disease-causing organisms). The noxious (unwanted) species may be Microscopic (e.g. bacteria, viruses, amoebic cysts, protozoan cysts) or Macroscopic (e.g. cockroaches, termites, mosquitoes or bats).

For example, it is known that exposure (irradiating) with ultra violet light (at sufficient flux density and appropriate wave length) will kill bacteria and inactivate many organisms or life forms by inactivation or destruction (disinfecting) of essential deoxyribonucleic acid (DNA), and/or ribonucieic acid (RNA) replication sequence/s. Exact details of such groups of noxious species can be found easily in publications released up to date (on the subject of waste water disinfecting) by the Water Environment Federations Research Foundation (WEFRF) and the Environmental protection agency (EPA). An example for such microscopic life forms may include spore forming or non spore forming or viruses or bacteriophage or cysts. Some examples are as follows:

### Non spore forming

Escherichia coli
Enterobacter cloacae
Direct Total Microbial Count groups
Fecal Coliform group
Aeromonas hydrophilae/suberia
Citrobacter freundii
Campylobacter jejuni
Thermotolerant coliform (groups)
Fecal streptococcus group
Heterotrophic ( plate Count group)
Klebisiella pneumoniae
Legionella dumofii
Legionella pneumophila
Mycobacterium avium
Staphylococcus aureus
Streptococcus faecalis
Salmonella typhi
Fecal streptococci / enterococci
Salmonella spp. group
Mycobacterium chelonae
Mycobacterium fortutuitum
Pseudomonas aeruginosa
Shigella sonnei
Total Coliform group
Yersinia enterocolitica
**Spore forming**
Bacillus subtilis
clostridia group
**Viruses / bacteriophage**
Coxsackievirus B-1 to B-5
Coxsackievirus A-9
Echoviruss 1
Echoviruss 11
H-1 parvoviruss
Hepatitis A virus
Human retrovirus type II
Simian rotavirus
B 40-8 bacteriophage / bacteriodes fragilis
F-specific bacteriophage
Somatic coliphage group
V1 bacteriophage
Polio-1
Polio-2
Polio-3
Reoviruss-1
Reoviruss-3
**Cvsts**
Criptosporidium parvum oocysts
Entamoeba histolytica
Acanthamoeba culbertsoni
Giardia lamblia
Giadia muris
Naegleria fowleri
Naegleria gruberi
**Macroscopic species**
Cockroaches
Termites
Mosquitoes
Bats

Current methods and means for disinfecting liquids or gasses using lamps or laser light sources are limited in their optical distribution scope or efficiency, as well as in their respective design geometry's - due to limitation imposed by their respective optical distribution architectures. The known existing methods and means are not using any optical fibers and crystals, or reflective end - cups interfaces, or semi holographic, partially dielectric rings, so they are limited in delivering simultaneously optical energy to a plurality of arranged points, across a predetermined dimension. These limitations impose restriction on the geometry's of the known methods by not having an adequate splitting, distributing, delivery and projection means. These limitation also prohibit the previous methods from creating, or facilitating optical distribution network for disinfecting liquids or gasses by using at least one central or remote light source. The present invention overcomes these limitations. Firstly, by using optical fibers for delivery and distribution and/or diffusing, and of lasing of radiation, further more, the ability of the present invention to deliver primary wavelength of optical energy via optical fibers for substantial distances for conversion at the end-cup crystal interface (e.g. such as in the present invention), by delivering one primary wavelength and converting said wavelength at the end of a fiber the present invention provide important inventive step by a) reducing the damage threshold at the point of entering the fibers by using a longer wavelength (e.g. such as a 1064nm wavelength in the IR., such wave length are known to be especially suitable for large distance transmission applications in IT and telecommunication optical distribution networks). Enhancing the delivery scope of optical fibers , eliminating the need to use expensive UV capable fibers such as HGFS (e.g. High Grade Fused Silica) which have only limited UV transmission capabilities. b) By having the ability to split the output of a single light source a cross tens, or hundreds, or thousands of points simultaneously (e.g. in remote locations, or remotely positioned projection, and/or diffusion points) substantially enhancing design scope of disinfecting reactors according to the present invention. C) The present invention is not so limited , that's way the present invention could be used in a wide range of disinfecting application including advanced integrated networks wherein the disinfecting processes occurs at a plurality of points of use, (e.g. such as taps) or at a central reactor (e.g. a conduit or a chamber) at a plurality of end user points of use. Further more the ability of the method of the present invention to split the laser beam into a plurality of substantially distanced points, facilitates transmission of adequate wavelength (e.g. sufficiently low wavelength) and frequencies of light for the production of Ozone (e.g. 03) wherein both designers and end user could benefit from safer geometry's having the ability to create a combined multi-processing network platform for disinfecting liquids and gasses.

Current methods and means for disinfecting liquids or gasses using lasers and lamps will be described in order to emphasize and indicate the novelty and inventive progress of the present invention.

References to previous patents, methods and means are forthwith included to indicate the inventive steps and evolutionary progress of the present invention.

The utilitarian scope of the present invention is embedded in a novel methodology wherein unlike previous methods and means, the present invention facilitate the furnishing of an interactive modular network of optical infrastructure for disinfecting liquids and gasses. Further more, the present invention facilitates interconnectivity and interoperability between producers and end users by utilizing the principle of single and/or bi-direction light transmission, harmonic conversion and/or frequency doubling. The ability of the present invention to split and guide light across a local, and/or large area network, is limited only by the efficiencies and/or tolerances such as damage threshold of the materials to be used. (e.g. such as coupling and transmission of the laser energy within the damage threshold of the fibers, the crystals, and light sources).

The present invention provides a methodology for delivering light at high intensities across such disinfecting network, wherein the light is of a particular wavelength (e.g. primary wavelength) and convert (e.g. 2nd, 3rd, 4th, harmonic generation) and/or alter said light at end user, and/or point of use into adequate wavelength maximizing delivery of a predetermined dose for effective germicidal effect harnessing transfer principle by total internal reflection of optical fibers inter-mated with crystals for the purpose of carrying to and/or from for real time networking between a plurality of environmental protection means (e.g. such as a plurality of disinfection reactors) in domestic, municipal, regional and international (e.g. in places wherein water treatment plants, or systems are positioned, and/or moved across geographically separated locations, or in neighboring borders to be positioned stationary, and/or mobile positioned on tracks and vehicles, or flown, or shipped, or put in oceanographic or space stations, across a plurality of remote destinations for disinfecting liquids or gasses using light which is harmonically delivered, and/or distributed, and/or frequency doubled and or diffused, and or projected via optical fibers.

The use of lasers as a source for UV radiation for disinfecting processes is known in the art. For example, the American patents US 466 1264, US 4816145, US4265747 and US 5364645, all of which disclose methods for disinfecting processes based on irradiation by a UV laser. However, all these patents disclose methods in which the light source itself, i.e., the laser, is attached, or in proximity, or integral to a conduit or chamber (e.g. such as in devices wherein the light source is integral to said conduit or chamber, and/or is directly positioned to the cross section, or flow direction, or against the flow direction). Such devices, according to previous methods and means require a complete unit each (e.g. including light source, its associated power supply unit, lenses, reflective mirrors or other optical surfaces for laser deflection,), making network of reactors, or remote disinfecting processes impossible economically (E.g. to disinfect three separated locations in a particular building on must have three light sources etc..)

The present invention is not so limited. That's way the present invention could be used for wide range of disinfecting applications in municipal, industrial, domestic, water and air recycling, in industrial cooling towers using liquids or gasses, or in paper, or computer chip manufacturing sites, in medical domain or in medical applications requiring selective medical preparations involving liquids or gasses, (e.g. blood, plasma, body fluids) in medical or surgical transplants, or in disinfecting air for hospitals, in air-conditioning systems, in the refrigerator industries, in the food & drink industries, in the semi-conductor, or other precision industries requiring clean rooms to a predetermined standards, biotechnology reactors having industrial photosynthesis capabilities, photosynthesis algae reactors lit, or powered, by global solar radiation (sun light which reached the earth surface and has been collimated by the earth atmosphere), in drinking water applications requiring the furnishing of an interactive optical fibers and crystals infrastructures or networks for disinfecting a plurality of rooms in a predetermined building making available for the 1st time, disinfecting optical - network of reactors driven by a single light source (e.g. such as a solid state laser), in agriculture or in drilling wells for water or petroleum, or gas, or combinations, in drilling applications under the surface of the sea beds or in under ground wells where the need to prevent clogging of under/above filters for liquids or gasses, as well as many types of mobile units for intervention task forces to disaster areas wherein damage to infrastructure support means have been caused by floods, hurricanes, storms, Vulcan activity such as earth quakes. Or in places where the ground water have been contaminated, or in places wherein medical aid is not adequate, or available and so local or regional population must equipped themselves to deal with the spread of diseases or liquids or gasses cross contaminated by noxious bacteria.

The present invention is bringing light using optical fibers and crystal interfaces into the liquids or gasses in a plurality of distanced locations from a centrally and/or remotely positioned radiation unit facilitating the furnishing of an interactive optical distribution network for disinfecting liquids or gasses as well as facilitating the design of semi-holographic disinfecting reactors (e.g. in or around a predetermined conduits or chambers).

The present invention provides the ability to split the output of a single laser beam (or light source output into tens, hundreds, or thousands of separated, individually positioned (e.g. fibers distributed within conduits or chambers or around a predetermined space or dimension) fibers and/or fiber bundles (terminated with crystals) - providing producers and end users in the field with unparalleled flexibility in designing reactors free of the limitations imposed by previous methods and means.

The present invention by utilizing fibers or fiber bundles is able to provide a plurality of points on a network (locally, or remotely spread to cover small or large areas) with adequate flux density simultaneously - for geometrically enhanced, more efficient, and/or more economical, requiring less maintenance, allowing the transfer and/or performance, and/or delivery of important data acquisition commands across the optical platform in variety of protocols to and/from plurality of operating reactors on a predetermined network (using optical fibers for carrying primary and/or secondary wavelength for disinfecting as well as the optical data from sensing peripherals making use of the same fiber and/or bundle to carry said signals back and forth from a remotely positioned control units, in real time. This provide for beneficial advantages in using a single centrally located light source for disinfecting a plurality of different location simultaneously. The present invention also present important safety by providing 100% electrical safety given the distance between the light source (centrally, and/or remotely located) and the emitting end, and/or side of the fiber, before or after said fiber has been terminated with appropriate crystal (for performing 2nd, and/or 3rd, and/or 4th harmonics on the primary wavelength originated in the central light source.

The present invention provide a novel methodology for creating an interactive disinfecting network using a single radiation unit bringing the light via optical fibers to a plurality of predetermined points simultaneously (e.g. such as remotely positioned, and/or separated locations, and/or rooms, and/or channels, and/or taps, and/or a plurality of remotely positioned conduits or chambers).

The ability of the present invention to deliver through at least one fiber, or fiber bundle (e.g. through a fiber network - a primary wavelength in the IR region and convert said wavelength at the end of the fiber by a crystal interface, to a lower wavelength (e.g. such as in the UV spectrum) provide for novel methodology wherein high powers could be coupled into the fibers given the longer wavelength (e.g. such as 1064nm). Compare to UV wavelengths which is substantially shorter and could put the threshold (e.g. damage thresholds of the fibers) under strain.

Currently used methods and means using lasers for disinfecting liquids and/or gasses, are limited in their efficiency scope and ability to provide economically sound, environmentally harmonious, geometrically efficient disinfecting reactors. Present methods and means fail to address the rising needs of today's increasingly stringent standards. Present methods and means using lasers are cumbersome, requiring the laser to emit light in the UV regions of the spectrum, such lasers are expensive, require the introductions of gasses (e.g. gas lasers, or eximer lasers), often requiring substantial amounts of periodical maintenance and/or replacements, present methods and means for using lasers for disinfecting liquids or gasses are geometrically limited, requiring the laser or radiation unit to be positioned in proximity to a conduit or chamber wherein the liquids or gasses are to be disinfected. Previous methods and means require large numbers of individual lasers (or light sources) units for disinfecting liquids or gasses in a plurality of remote locations simultaneously.

The present known Ultra Violet disinfecting methods and the means which are used today for disinfecting water (references: Water Environment Research foundation WERF "disinfection models, principle components in UV disinfecting system design" 1997 in water disinfection) mostly using a large number of individual UV lamps (e.g. such as mercury arc lamps) which are arranged into banks of lamps. and inserted into the water creating a cumbersome and often large dimension for reactors, requiring higher levels of periodical maintenance, and/or requiring large space for installations sites. These lamp based disinfecting reactors being of large dimension and weight are often limited in their mobility scope, and are not available for large throughput application as mobile units. These lamps are at present the principle means of generating CW (Continuos Wave) UV energy used for disinfecting Further more, These lamps are polychromatic lamps where by up to 85 % of the light output is monochromatic at a wave length of about 254nm which is "considered" to be within the optimum range about 250nm to about 280nm for germicidal effects to take place (e.g. selective inactivation of essential DNA and RNA replication sequences) these present methods and means are expensive requiring large amounts of lamps in a single system, these lamps require periodical cleaning (from colloidal deposits or hard water deposits) using expensive chemicals (e.g. soap or acidic compounds) causing system down time and high cost maintenance. These lamps (which are used at present) are rigid (inflexible) are immersed in water channels in large numbers to form a banks of lamps and are often causing head loss (e.g. places within a conventional UV disinfecting system geometry whereby water are passing above or below a bank of lamps without being disinfected) and reduction in the efficiency of presently used systems, forcing designers to reduce the flow rate through a particular channel and split it over a number of channels. Present methods and means currently used to disinfect water (or air) (e.g. liquids and/or gasses) often require complex and costly mechanical and hydraulic means to lift the lamps in and out of the water, to operate and/or activate brushes on the lamps protecting sleeves (e.g. normally made out of quartz) periodically, or continuously (for cleaning colloidal deposits and/or hard water deposits), further increasing maintenance costs and increasing the energy consumption of currently used UV disinfection reactors (systems). Further more, system based on UV lamp technology often require quartz sleeves or other specially formulated transparent protecting tubes or envelopes for protection of the lamp in the water (liquids or gasses) while being able to transmit UV throughout. These sleeves often crack from hot spots, generated by the polychromatic characteristic of these lamps (often mercury based lamps, having sufficient IR radiation in their output to cause heating, heating is not uniform due to uneven coverage of colloidal deposits, and or hard water deposits on the surface of the sleeve/s) endangering producers or end users as well as the environment It is known that illuminating a transparent or opaque surface with ultra violet light energy (at the appropriate wave length and sufficient flux density) will penetrate through the surface and according to the nature of that material, that ultra violet light energy would be absorbed into the underlying material, an example of such process may be: inactivating of DNA and RNA replication sequence/s by a CW (Continuos Wave) light from a UV microwave excitation, lamp of microorganisms until the flux density of the penetrating light is diminished or absorbed by the microorganisms themselves (photochemical damage to RNA or DNA within the cells of an organism is due to the nucleic acids being most important receptive absorbers of light energy) or by presence of suspended materials, or organic or non organic surrounding compounds or materials to below the threshold required for effecting disinfection.

The efficiency (factor) of devices which disinfect by using ultra violet light is normally restricted by the depth of penetration (UVT or the UV transmission throughout that material) of the ultra violet light into the material to be disinfected. This factor limits the flow through cross section of the conduit through which material being disinfected must pass. (This factor also prevents ultra violet light from being used to disinfect opaque substances, or limits the scope of efficiently distributing UV light in order to reach remote location in industrial or domestic environments whereby space is at a premium).

The method of the present invention and device according to it eliminates these efficiency limitations. The method of the present invention facilitates the use of a central radiation unit, having a high intensity source of light wherein the light from said radiation unit is distributed, or delivered, or diffused or combination at the point of use (e.g. such as end user taps), unlike previous methods which bring the liquids or gasses to the light (e.g. liquids or gasses are made to pass through the light), the present invention bring the light to the liquids or gasses to be disinfected. Further more the present invention facilitate the formation of a novel interactive network of optical fibers and/or crystals delivering optical energy (centrally positioned laser) having a primary wavelength in the NIR, or IR region from about 800nm to about 2400nm to a remote location, the primary wavelength is converted using crystals (e.g. such as a KTP, or PPKTP types) which generates 2nd, or 3rd, or 4th, harmonics on said primary wavelength (e.g. such as on 1064nm)

Therefore, the device of the present invention may be applied to the disinfecting of divers substances; such as air, water,(e.g. for drinking, washing, or irrigation ), drinkable liquid (e.g juice, milk or vinegar), filterable food (e.g. baby food, ketchup, or jam), medical preparations, surgical transplants, cosmetics, sewage, waste water, sea water and the like.

Further more the device of the present invention is especially adaptable for installation into filtration units (such as those which are used for the filtering out of particulate or suspended materials (suspended solids) from any of the above mentioned substances) this special adaptation results from the device of the present invention distributing the (e.g. ultra violet ) light through side emitting optical fibers; wherein these fibers are easily integrated into the porous screens or surface disks or membranes or magnetic elements used for filtering.

Another example of lighting causing disinfection relates to illuminating a volume with visible light, in order to disturb the breeding cycle of cockroaches. This type of disinfecting is especially applicable to sewers, food storage areas, and hollow sections of building members (e.g. in attic spaces, or in conduits for wiring or plumbing) .

In principle, each noxious species is disturbed or destroyed by some frequency (at the appropriate wave length and flux density) of light.

Thus, since optical fibers (e.g. end glow types and/or side emitting) exist for the transfer and/or delivery, and/or diffusions of at least one or more wavelength and frequencies of light from about 180nm to about 2400nm, the benefits of disturbing, or destroying, or equalizing noxious species (in confined and predetermined spaces) may be achieved using the devices according to the present invention. This is an Environmental benefit, since on a species specific basis, all other known methods require the introduction of toxic substances such as chlorine or chlorine compounds (e.g. hypochlorous acid or HOCI, hypochlorite ion OCI- or monochloramine, insecticides, pesticides, etc.) which over certain periods of time produce accumulating 1st and 2nd generation compounding volumes which are endangering man kind and its environment.

US-A-5262066 discloses a process for treating fluids, wherein a UV radiation source and optical fibres are employed.

### SUMMARY OF THE INVENTION

The present invention relates to a method for remote disinfecting liquids and gasses comprising; distributing at least one optical fiber in the region containing the liquids or gasses to be disinfected; aligning at least one radiation unit having a high intensity source of light into said fibers and radiating said liquid or gasses by said optical fiber over a predetermined period of time, wherein said radiation unit is a laser and wherein the primary radiation frequency of the laser is converted to another secondary frequency, before or at the disinfecting site, such that the secondary radiation emitted from the optical fibers has a different frequency from the primary laser frequency.

The present invention relates further to a device for disinfecting liquids and gasses by the above method comprising ; a conduit or a chamber containing the liquids or gasses to be disinfected, at least one optical fiber distributed within or around or integrated into the walls or the body or the vicinity of said conduit or chamber, and a laser having high intensity source of light aligned into said fibers wherein at least one crystal interface is attached or integrated to at least one optical fiber or bundle of fibers end termination for harmonically converting the wavelength of the incoming primary pulse to a lower wavelength of outgoing pulse.

For the purposes of the present invention, a "side emitting optical fiber" is any optical fiber which will transmit a desired frequency of light from one end to the other (using internal reflection of the fiber), and simultaneously allows some portion of the transmitted light to escape from the fiber during the transmission path along the length. This escape of light may be continuous along the entire length of the fiber, or may be restricted to a plurality of ("exposed") locations along the fiber. (examples of such fibers may include high grade fused silica, silica, ,plastic optical fibers POFs, polymer matrixes, anaerobic non toxic liquid light guides).

In the context of the present invention "a porous screen" relates to any passive or active element in a filtration system whose function is to bar the passage of particulate above or below a predetermined size. Today, filtration screens come in many topological configurations; including flat panels, stratified aggregate beds, thin or thick surface disks, perforated cylinders, magnetic elements. A settlement tank, even though it may be used for the removal of particulate, is considered a conduit or chamber and not a filtration unit (for the purpose of the present invention). In the context of the present invention, a plurality of end emitting (optical) fibers may be substituted for a side emitting (optical) fiber; when the aggregate surface area of the ends of the end emitting fibers is approximately equal to the operational (exposed) surface area of a side emitting fiber; or when the flux density at the appropriate wave length of the delivered light can be otherwise equalized and simultaneously broadly distributed spatially.

In the context of the present invention ultra violet radiation is optical radiation for which the wavelengths are shorter than those for visible radiation, < 400nm (UVA 320nm - 400nm, UVB 280nm - 320nm, UVC <280nm).

In the context of the present invention visible radiation (illumination) is any optical radiation capable of causing a visual sensation directly, 400nm - > 700nm.

In the context of the present invention uniformity is a measure of how the irradiance varies over a selected or predetermined area (e.g. trance uniform flux density)

In the context of the present invention sun light terrestrial spectra is the spectrum of the solar radiation at the earth's surface, wherein direct solar radiation is the part of extra terrestrial solar radiation which, as a collimated beam/s, reaches the earth's surface after selective attenuation by the atmosphere.

The term "conduit" in the present invention is referring to any space having inlet and outlet openings (e.g. pipes or window frame)

The term "chamber" in the present invention is referring to any space which has one opening (e.g. a container or a storage tank)

In the context of the present invention a "conduit" relates to a predetermined volume such as a chamber which is a closed volume having an opening, a conduit (as its normally understood) meaning a closed volume having an entrance and an exit, a connector meaning a closed volume having multiple openings, a closed volume having only microscopic opening or any combination thereof.

In the context of the present invention a plurality of liquid light guides may be substituted for side emitting ( optical) fibers ; when the aggregate surface area of the end of the liquid light guide is approximately equal to the operational (exposed) surface area of side emitting fiber, or when the flux density at the appropriate wave lengths of the delivered light can be otherwise equalized and simultaneously distributed spatially, or uniformly or a combination thereof (e.g. high grade fused silica and/or liquid light guides having transparent or semi transparent sleeves).

In the context of the present invention a plurality of side emitting optical fibers may be grouped together in bundles having a common end termination at one end and individual fibers end portion (end termination) at the other, or the may be looped to a single common end termination or splits (multi-track bundle type, e.g. single input multiple individual outputs) formation bundles (distributed within or around or integrated to the walls of a conduit or a chamber), (random, rectangular, circular etc.) wherein predetermined species specific optical dose delivery (flux density required for disinfecting) is maximized for a predetermined radiation unit of a specific spectral distribution.

In the context of the present invention a conduit is any predetermined space with one or more opening for the liquids or gases to go into or through or out of said conduit.

In the context of the present invention a chamber is any predetermined space having one opening for the liquids or gases to be put - in or out - of or stored or held in (temporarily or permanently) said chamber.

In the context of the present invention peak power is the top most powerful point generated in a predetermined single optical pulse over predetermined time of its duration.

In the context of the present invention pulse duration relates to the over all predetermined time span which constitute a single optical pulse over predetermined time. (e.g. nano-second often written in short as Ns, Pico second Ps, fem. seconds in short as Fs etc.)

In the context of the present invention pulse repetition rate relates to the number of pulses generated over predetermined time (e.g. pulse per second, normally measured in Hz, for example from about 10Hz to about 18,000 Hz). Further more , in the context of the present invention said pulses are launched into optical fibers each having at least one crystal and/or lens at its end termination interface.

In the context of the present invention pulse wavelength relates to the specific wavelength in which a predetermined pulse is being generated, or projected, wherein pulses so generated within the laser could have different wavelength when exiting or projecting from the fiber (or bundle of fibers) end termination/s, through the crystal interface. (e.g. holographic element)

In the context of the present invention wave length range relates to the range of wave lengths which a predetermined monochromatic or polychromatic light source or any combination thereof produce. Further more the resulting range of wave lengths transmitted or dispersed or emitted or irradiates or illuminates or refracted or reflected or any combination thereof through (at least one) predetermined optical fiber or bundle/s.

A utilitarian utilization of a predetermined wave length, or beneficial spectrum - specific context or range or band or plurality of wave lengths or the associated cut - of frequencies from groups of predetermined transmitting materials, or substances associated cut of frequencies of light, these parameters relates to at least one CW or PW E.g. Continues Wave or Pulsed Wave light energy source outputs or inputs wherein or exited or lase or harmonically generated by at least one crystal or filtered or any combination thereof into a predetermined volume of liquids or gasses therein (within the conduit or chamber).

In the context of the present invention pulse modulation relates to the amount or type or timing or combinations of a predetermined modulation (e.g. control changes or timing changes or frequency changes or level changes or any combination thereof) applied to a specific pulse or sequence of pulses before or during or after or in accordance with the process or plurality of pulse generation processes used in a specific light source or sources or their associated control electronics and integral or non integral power sources.

In the context of the present invention down conversion relates to any optical process or plurality of processes in which a primary higher wave length pulse is converted (e.g. down converted to its harmonics) to a lower wave length or harmonics by the use of a predetermined crystal or any predetermined optical element (e.g. this process often called harmonic generation for example a 2nd harmonic on a 1064nm [IR] pulse will be 532nm [Vis] etc.

In the context of the present invention 2nd, or 3rd, or 4th harmonic generation also relates to inter-cavity (e.g. inter-cavity) harmonic generation processes which occur within a predetermined laser cavity by at least one integrated, or externally attached, or supported crystal, or plurality of aligned, or coupled sequentially interconnected crystals (e.g. when the light source is a laser).

In the context of the present invention triggering means activating or deactivating or controlling or any combination thereof of parameter or plurality of parameters related to the creation and generation of a specific predetermined pulse transmitted in real time through at least one optical fiber (or any predetermined sequence or plurality of sequences of a predetermined number of pulses) over predetermined time. (throughout the fibers).

In the context of the present invention up conversion relates to any optical process or plurality of processes in which a primary lower wave length pulse is converted (e.g. up converted to its harmonics through excitation and the use of crystals) to a higher wave length or harmonics by the use of a predetermined crystal or any predetermined additional optical element or material in a given state (e.g. liquid, solids, gasses) having the inherent ability to be excited. This process often called harmonic generation and is performed with the aid of a predetermined crystals or additional optical elements, e.g. SHG, THG, FHG, etc.)

In the context of the present invention a crystal end-cup is any crystal attached or integrated, or integral, or supported by, or positioned in proximity to the end termination of the fiber for efficient 2nd, and/or 3rd, and/or 4th harmonic generation from it (e.g. for illumination or irradiation of a predetermined volume of liquids or gasses in the conduit or chamber)

According to the preferred embodiment of the device of the present invention, the device includes having a conduit or chamber wherein the fibers are distributed and/or includes having means for supporting or maintaining the distribution of fibers within the conduit or chamber.

According to one especially useful embodiment of the device of the present invention, the light is primarily ultra-violet, for the disinfection (selective inactivation or destroying) of bacteria or other microorganisms life forms.

Light from the radiation unit is aligned into the optic fiber by optically aligning a beam of light from the radiation unit into an end termination (end portion) of said fiber, or by integrating the radiation unit into the fiber, or by optically aligning beams of light from the radiation unit into (through) the side of the fiber.

According to the preferred embodiment of the device of the present invention, the conduit or chamber is a filtration unit having at least one porous screen or (element) surface disk for the removal of particulate material. Further more, the optical fibers may be integrated into or onto at least one of the screens or surface disks (e.g. filters elements).

There may be Environments which cause deterioration to the surface of the optic fibers This deterioration may be by reason of physical contact (such as the high velocity impacting of particles onto the fiber's surface) or by reason of chemical reactivity between the fiber's surface and liquids (or gases) in the conduit or chamber.

in such cases it is advised to isolate the fibers from their operating environment by using a transparent or opaque sleeve. Thus, one embodiment of the device of the present invention has a transparent or opaque sleeve enclosing the optic fibers, while in another equally useful embodiment the sleeve is integrally enclosing the optical fibers.

According to another useful embodiment of the device of the present invention, the light is primarily visible, especially for disturbing the breading cycle of cockroaches.

The selection of light source (for the production of visible light and/or ultra violet light and/or other wave lengths and frequencies of light ) is according to the target species and to their wave length and frequency specific light sensitivity. For example, according to a novel embodiment of the device of the present invention the conduit or chamber of the device (or the conduit or chamber wherein the fibers of the device are distributed) is a sewer pipe, a section of sewer pipe, or a network of sewer pipes.

According to this embodiment, bacteria and/or cockroaches and or other noxious species may be eliminated from (disinfected e.g. selectively inactivated or destroyed) the sewage prior to (or during or after) standard treatment and/or discharge.

This treatment of the sewage while still in the sewer pipe network is especially useful for improving public health (a) in large urban areas where many regions of the sewage collection network are distant from their eventual treatment plants, (b) in regions where the eventual sewage discharge is through a cesspools (distributed local ground seepage networks) and where simultaneously the ground water level is high, and (c) in urban areas where the human population does not have proper access to modern medical care (and is thus subject to epidemic noxious infestations), (d) intervention task forces to disaster areas and regions which may experience floods, typhoons, hurricanes or earth quakes and are therefore in need of urgent disinfecting of liquids or gases due to collapsed or damage to existing infrastructure (e.g. water pipes and treatment plants, important air passages), (e) sea water filtration systems, (t) pre/post filtration for sterilization (g), pre/post disinfecting of industrial water recycling .

Another useful embodiment of the device of the present invention relates to the conduit or chamber (wherein the fibers are distributed) as a closed space. For example, when the closed space (conduit or chamber wherein the fibers are distributed and /or are supported) is (a) the aeration volumes of loosely packed soil, (b) a cabinet, (c) a closet, (d) the space below a raised floor, (e) the space above a drop ceiling, (f) the space in a hollow wall, (g) an attic, (h) a crawl space, (i) the space between stored articles, (j) the space between infrastructure support connections (e.g. underground, electric or telephone cables), (k) a water carrying pipe or module, (1) a shoe (when not being worn), the space in a brush head for cleaning conduits or chambers, (m) a window frame to the open air, (n) a tunnel, (o) oxygenation and water treatment ponds, (p) the head of a tooth brush, (q) a vacuum cleaner attachment.

Many of the embodiments of the device of the present invention are functionally more efficient when they have a computer controlling the output of the radiation unit. The computer may effect this control by regulating the electric current powering the radiation source (for certain type of light sources), may effect this control by regulating the alignment of light from the radiation unit into the side emitting optical fibers, or by regulating the reflective feed back of the terminal end cap at the far tip of the side emitting optical fibers.

According to the preferred embodiment of the device of the present invention, the radiation unit is a laser. According to a further refinement of the preferred embodiment of the present invention, the optical fiber's path in the conduit predetermined space is arranged for the production of at least one region of constructive interference in the conduit wherein liquids or gases are present. (and this result is most conveniently accomplished when the radiation unit is a laser).

This constructive interference is easier to accomplish in a controlled geometry when the fiber's path (according to the present invention) is of a spiral (or zig-zag) arrangement for the forming of a plane, a cone, a cylinder, or a smooth surface.

Another mutually compatible method wherein the constructive interference is accomplished arranges the path of fiber with the fiber bent back along its own path to form at least one section of parallel fiber path.

A further mutually compatible method for use of the device of the present invention wherein the constructive interference is accomplished has a reflective member parallel to at least one section of the fiber in the conduit or chamber.

According to the preferred embodiment of the device of the present invention, this reflective member is integral to at least one fiber.

According to another useful embodiment of the device of the present invention, the end of the optical fiber or fibers in the conduit or chamber are arranged for the production of at least one region of constructive interference in said conduit or chamber ( when the radiation unit is a laser). The preferred method for accomplishing this effect according to this embodiment has the end of at least one of the optical fibers placed opposite a reflective member.

According to another novel embodiment of the device of the present invention, a holographic optical element (e.g. a computer generated film, digitally encoded memory chips or disks) is incorporated into the means for aligning the sheath of the fiber (in the conduit, a chamber) for the production of at least one region of constructive interference in the conduit or chamber (when the radiation unit is a laser).

For example, according to one method for use of the device of the present invention the holographic element is aligned between the radiation source and the end of the optical fiber, or according to another method for the device of the present invention, the holographic element is located in the conduit or chamber and aligned at the terminating end of the optic fiber. The use of holographic elements especially outside of the visible spectrum (such as in many applications of the device of the present invention) is forming an invisible hologram (e.g. uv hologram within the conduit or chamber).

According to another interesting embodiment of the device of the present invention, a reflective end cap affixed on the terminal end of the optical fiber for the production of at least one region of constructive interference in the conduit or chamber (when the radiation unit is a laser).

According to another novel embodiment of the device of the present invention the disinfecting holographic element is formed with in a conduit or a chamber using at least one (side emitting) optical fiber wherein said holographic formation is originated out of at least one single wave length coherent light beam at about 187nm to about 320nm wherein focusing optics are used for conditioning the light beams (from the radiation unit) on entrance or exit to/from said fiber.

A novel environmental embodiment of the device of the present invention wherein a plurality of fibers are aligned to at least one radiation unit having a high intensity light source and said fibers are distributed within a conduit or a chamber wherein the fibers are grouped by a common end termination at one end, and wherein a plurality of their second ends portions are grouped together to form a brush, light from the light source is illuminating outwardly from the fibers sides and end portions for illuminating a predetermined volume of liquids or gases therein (e.g. internally within the brush head or externally within an external conduit or chamber).

According to a novel embodiment of the device of the present invention wherein a plurality of (side emitting) optical fibers end portions are grouped and harnessed to a common end termination within a modular attachment of a vacuum cleaner wherein the fibers other end portions are grouped together in the shape of a (fiber) brush, a radiation unit having a high intensity source of light and said light is aligned into said fibers, wherein the fibers are distributed within the conduit or chamber for illuminating a predetermined volume of liquid or gas therein.

According to a novel environmentally beneficial embodiment of the device of the present invention at least one high powered ultra violet laser is used in at least one pulsed mode or continuing mode (pw, cw) or combination thereof and said ultraviolet pulses and continuous waves are referenced, controlled and triggered by an accurate clock for illuminating a pre determined volume of liquid or gas therein (within a conduit or chamber).

According to a novel embodiment of the device of the present invention light from at least one light source is aligned to at least one multiplexed, multi-dimensional distributed layer of side emitting optical fibers for the purpose of removing or self cleaning (by photons optical impact) the colloidal deposits and/or hard water deposits from immersed fiber/s sleeves and optical outputs or reflectance member or conditioning optics or light guides or a combination thereof providing enhanced ultra violet transmission from about 180nm to about 280nm within the conduit or
chamber - ensuring that the UV dose delivery is calibrated against species specific calibration standards for (adequate) illuminating or irradiating (disinfecting) of a predetermined volume of liquid or gas therein (within the conduit or chamber).

According to a novel embodiment of the device of the present invention, at least one region of constructive interference (holographic) at the preferred wavelength in the range from about 180nm to 270nm and flux density is
calibrated against species specific calibration standards wherein DNA or RNA replicating sequence of at least one micro-organism or at least one macro organism or a combination thereof is selectively inactivated.

According to a novel embodiment of the device of the present invention, at
least one high intensity light source is positioned at a predetermined distance from the conduit or chamber wherein the light is guided or launched or projected or trajected or transferred or diffused or a combination thereof by at least one side emitting optical fiber for the production of at least one region of constructive interference within said conduit or chamber but do not physically restrict (water) or gas movement or flow - in, at, out or through said conduit or chambers cross sections.

According to a novel environmental embodiment of the device of the present invention wherein the fibers are distributed within and around a plurality of optically transparent (water) filtering disks, screens for simultaneously filtering suspended solids in a conduit or chamber while illuminating or irradiating a pre determined volume of liquid or gas therein.

According to a novel embodiment of the device of the present invention wherein at least one radiation unit having a high intensity source of light and said light is pulsing or continuous or a combination thereof (at about 180nm to about 400nm) is aligned to a plurality of side emitting optical fibers and said fibers are distributed within a conduit or a chamber for illuminating a pre determined volume of liquid or gas therein, wherein an adequate level of particle size distribution (PSD) is reached (by pre filtering or recycling of said liquids or gases) to ensure continues (adequate) transmission of optical radiation of a specific spectral distribution over predetermined exposure time (e.g. disinfecting dose).

According to a novel embodiment of the device of the present invention the radiation unit having a high intensity source of light is a fiber laser wherein said fiber laser could ergonomically integrate into a tight space inside or outside of a conduit or a chamber.

According to a novel embodiment of the device of the present invention the conduit or chamber is a filtering unit having at least one magnetic element or field for filtering (out) of metallic substances or compounds (within the liquids or gases to be disinfected).

According to a novel embodiment of the device of the present invention wherein conditioning optics (e.g. a lens, a beam spliter, an acousto-optical shutter, a shutter, a focusing lens, a beam expander, a beam expander telescope) are used to condition beams of light (from the radiation unit) on exit from the fiber.

According to a novel embodiment of the device of the present invention an of axis beam or reference beam is projected from end portion/s of at least one side emitting optical fiber wherein said fiber/s end portions are distributed within a conduit or a chamber for the production of at least one region of constructive interference (within the conduit or chamber)

According to a novel embodiment of the device of the present invention an ultra violet (invisible) hologram is formed within a conduit or a chamber out of at least one region of constructive interference and said ultra violet hologram is aiding to the elimination of head loss within said conduit or chamber geometry (without distracting the liquids or gases flow within).

According to a novel embodiment of the device of the present invention the level or flow rate of liquids and gases within a conduit or a chamber is calibrated to a radiation unit for saving on energy consumption or maximizing the efficiency of illumination or irradiation of a predetermined volume of liquid or gas therein (within the conduit or chamber).

According to a novel embodiment of the device of the present invention the conduit or chamber (wherein the fibers are distributed) is the water or air systems in a car (e.g. car air condition, radiator, water system for cleaning the car windows).

According to a novel embodiment of the device of the present invention wherein the conduit or chamber (wherein the fibers are distributed) is the water or air (liquid or gas) systems within a public transport train or buss.

According to a novel embodiment of the device of the present invention where by a high intensity IR pulsed light source launches pulses at a repetition rate from about 10Hz to about 1Ghz at a pulse width from about 118Ps to about 100ns having a wavelength from about 850nm to about 2400nm wherein these pulses are launched into single mode fibers where by a connected or integrated crystal is aligned at the end point of said single mode fibers (end termination entry into the reactor active cross sections) for creating 4th, or 3rd, or 2nd harmonic generation or any combination therein for illuminating or irradiating a predetermined volume of liquid or gases within the conduit or chamber with a wide range of spectral signatures.

According to a preferred embodiment of the present invention wherein at least one fiber is a PM (Polarization Maintaining optical fiber) type fiber having its polarity calibrated against the polarity of the crystal or plurality of crystal interface maximizing conversion efficiencies along a predetermined optical axis, or in the direction of the optical path or in a predetermined conduit or chamber geometry's (e.g. a predetermined optical fibers, crystals and lasers disinfecting reactors.

According to a novel medical embodiment of the device of the present invention wherein a predetermined conduit is used for external dialysis processes and wherein said conduit around which the fibers are distributed and/or supported is illuminating or irradiating a predetermined volume of liquids or gasses therein. Further more liquids or gasses could be illuminated or irradiated in wide ranging medical treatments some which may include: 1) Medical preparation or medicines, 2) Medical discharges (e.g. during operations or other procedures, 3) During laser operation in invasive medical procedures, 4) During birth procedures for general hygiene, 5) In certain dentistry or orthodontist procedures and treatments, 6) In certain darmatological treatments 7) In disinfecting the air or water supply to and from different hospital patients. 8) During infusion procedures of liquids or gasses, 11) During transplant operations, 12) To medical transplants 13) Incubators for young or prematurely born babies. 14) In clinics or laboratories liquids or gasses main supplies. 15) In aquariums or other life preserving conduits or chambers, 16) In emergency equipment for resuscitation 17) In a village or city main supply of liquids or gasses to hospitals or domestic or industrial end users, 18) In clean rooms or environments used for diagnostics or medical treatments against noxious species

According to a novel utilitarian medical embodiment of the device of the present invention wherein small or large medical instrumentation can be disinfected efficiently using the device of the present invention. More specifically a conduit or a chamber wherein the fibers crystal end termination are distributed or supported according to the present invention could serve as or be integrated into or be in a proximity of or connected to or operating with or any combination thereof wherein predetermined pulse peak powers or pulse frequencies are utilized for specific equalization or inactivation of noxious species in : a) disinfecting tank or area or chamber, b) a working area having liquids or gasses in its proximity, c) a stove or table surface, d) a window frame or air passage, e) air-conditioning systems or air pumps, f) laundry areas and storage containers, g) semi-conductor manufacturing sites or rooms, h) laundress, i) food storage tanks or containers or rooms, j) drinks and beverages manufacturing conduits or chambers, k) conduits or chambers for dissemination of sea water, l) biologically controlled environments such as laboratories and their associated storage conduits or chambers in biotechnology industries. m) conduits or chambers used for the milk industry and related food produce industries n) Hygiene or educational predetermined areas or surfaces wherein contamination of bacteria occurs in conduits or chambers or in confined predetermined containers storage areas, o) In conduits or chambers for the preparation of baby foods or drinks, p) In medical emergencies wherein a need arise for immediate disinfecting of a predetermined conduit or chamber or surface relevant foe specific medical operation or procedure, q) In beds or mattresses wherein a predetermined conduit or chambers are to be used for transferring or distributing or disinfecting liquids or gasses therein, r) in liquids or gasses "at source" point of origination, s) In paper factories, t) in swimming-pools, w) In industrial recycling of liquids or gasses, x) In air treatment plants, y) In critical air passages, z) In the end user point of use of drinking water, z/a) In the end user point of use of air or other relevant unharmful gasses during medical treatments and procedures.

According to a novel environmental embodiment of the present invention wherein a high intensity pulsed light source is used down or up converted (e.g. 2^{nd}, or 3^{rd}, or 4^{th} harmonics and/or excitation, and/or pumping architectures or any combination thereof launched at the input of the fibers) at the other end of the fiber harmonically processed, or converted, or excited, or any combination thereof for illuminating (400nm to about 700nm visible spectrum) or irradiating (850nm to about 2400 and from 200nm to about 400nm) a predetermined volume of liquids or gases within the conduit or chamber (e.g. within active reactor geometry, or holographic type ring, or slits, or bar, or any combination of arrangements thereof).

According to novel environmental embodiment of the device according to the method of the present invention wherein the high intensity light source is a pulse laser which is used to provide primary pulses in the infra red (IR) portion of the spectrum, these pulses are being launched or distributed or transmitted through single mode fibers, or multi-mode fibers, or gradient index fibers, or light conducting layers, or any combination thereof to be converted or harmonized to produce UV (A, B, C) or visible or IR for illuminating or irradiating a predetermined volume of liquids or gases therein (E.g. within the reactor; a conduit or chamber).

According to a novel embodiment of devices according to the method of the present invention wherein light from a pulsed laser (e.g. high intensity, high energy, high peak power pulsed laser light energy) is converted into 2nd harmonic, or 3rd harmonic, or 4th harmonic (e.g. SHG, THG, FHG, etc.) generation processes using a predetermined primary wavelength according to a predetermined lazing material, or mirrors, or prisms, or lenses or any combination thereof (e.g. crystal positioned in a predetermined angles). Further more an especially beneficial embodiment of devices according to the method of the present invention is having said harmonic generation processes performed within a predetermined area of the cavity (or cavities) in a predetermined laser light sources geometry, prior to delivery of said high intensity or high peak power laser pulses into at least one optical fiber or bundle of fibers (e.g. using inter-cavity SHG, THG, FHG), for efficiently illuminating or irradiating a predetermined volume of liquid or gasses therein (within the conduit or chamber).

A novel embodiment of devices according to the method of the present invention is having at least one flash lamp as the primary pumping (e.g. optical pumping) of the laser (e.g. lazing rod) light source, a further beneficial method according to the method of the present invention is having at least one diode as the pumping (e.g. optical pumping) means of the high peak power light source.

A novel environmentally beneficial embodiment of the device of the present invention wherein the primary (e.g. pumping) wave length is selected from about 1064nm for Nd:yag/1064nm or GaAS/810-905nm or any combination thereof for efficiently illuminating or irradiating using crystal interface to generate 2nd or 3rd or 4th harmonic therein (with the light) wherein a predetermined volume of liquids or gases therein is adequately disinfected using short pulses having high peak powers in contradiction to CW average power which is generated by UV lamps (currently the principle means of generating UV energy for disinfecting).

A novel environmental preferred embodiment of the device of the present invention wherein the pumping wave length is selected from a predetermined electromagnetic spectrum wherein each laser according to the lasing material is pumping appropriate crystals for generating 2nd or 3rd or 4th harmonic or any combination thereof wherein the pick power of the primary or harmonic pulses provide for efficient illumination (from 400nm through to about 700nm visible spectrum) or irradiation (from about 220nm through to about 400nm for UV radiation) (from 700nm through to about 2400nm for IR radiation) of a predetermined volume of liquids or gases therein providing an adequate dose of pulses peak powers to efficiently inactivate DNA and RNA replication sequences.

A novel utilitarian embodiment of the device of the present invention wherein the conduit or chamber inner surface is grooved, or bent, or deposited or extruded or injected or glued or inserted or extended or pulled by variety of fashions, or coated spirally or deposited in layers, to extend inwardly in a predetermined patterns for the purpose of guiding hydraulically or pneumatically a predetermined volume of liquids or gasses therein, more specifically these extensions can take the shapes of (A) A spiral extending to cover at least one portion of the predetermined inner space along the length of the conduit or chamber - slowing or speeding the movement of the liquids or gasses throughout or (B) it can take the shape of a grid equalizing throughout while said predetermined liquids or gasses permanently or temporarily standing or stored therein, or in transition in and throughout said conduit or chamber or any combination thereof, wherein the fibers or wave guides are distributed for effective illumination or irradiation of predetermined volume of liquids or gasses therein with the light over predetermined time

A novel preferred embodiment of the present invention is especially useful wherein the conduit or chamber are integral to a vehicle, further more the engine or power source of the vehicle could serve or be diverted for operating the pulse or continuos laser source or pump or water or air flow or any combination thereof, further more, said engine could operate a single platform in which the flow rate or velocity or pressure or quality of liquids or gasses will control the pulse width or pulse duration or pulse peak power, or pulse wave length or pulse repetition rates or any combination thereof.

A further useful embodiment of the device according to the present invention is having a vehicle equipped with a high intensity pulse laser light source connected to batteries or the engine of the vehicle itself for illuminating or irradiating a predetermined volume of liquids or gasses therein (e.g. in the integrated conduits or chamber).

A preferred embodiment of the present invention wherein pulse repetition rate (1) or pulse duration (2) or pulse peak-power (3) or pulse- wavelength (4) or pulse - width (5) or any combination thereof are synchronized or locked to or referenced or timed or triggered or controlled or modulated by (when connected to a computer) software or hardware or any combination thereof.

Further more when water flow (6) or air flow (7) rates (e.g. liquids or gases) or any hydraulically or pneumatically aspects or parameters relating to the flow of liquids or gasses therein (in the conduit or chamber) or any combination thereof - these parameters are linked to reference (8), or to activate (9), or to control (10), or to modulate (11) a predetermined variety of control parameters from the active light sources power unit (12), or its associated control electronics (13) for generating high peak power pulses (e.g. harmonic generation by attached or integrated crystals into fiber end termination) or for the production of timing variation (14) or sequences of variations (15) in pulse duration (16), or width (17), or repetition rates (18), or peak power (19), or pulse wave length (20), or angle (21), or any combination thereof when the attached or integrated crystal (22) is positioned inside a ring (23) or rod (24) or pipe (25) or network of infrastructure support means (26) or flat or curled or twisted or pressed surface or any combination thereof. Further more an especially beneficial embodiment of the present invention wherein a plurality of parameters are software controlled for monitoring such as TSS (26a)(Total Suspended Solids) levels or Turbidity levels, (26b) or PSD (26c)(Particle Size Distribution) levels, or liquids or gasses flow rate (27) or any combination thereof (within the liquids or gases to be disinfected) are detected (28) in real time and/or synchronized (28a), to ensure continuos (29),(adequate) transmission of optical radiation (30) of a specific spectral distribution (31)over predetermined exposure times (32) e.g. disinfecting dose using the peak powers (33) of pulses or sequences (34) of pulses.

A novel utilitarian especially beneficial embodiment of the device of the present invention wherein a plurality of predetermined (variables) parameters or aspects or levels or time positioning of any combination thereof relating to the laser pulse generation means (e.g. high intensity pulse laser light source) and said parameters and aspects are interacting in real time for the purpose of illuminating or irradiating a predetermined volume of liquids or gasses over predetermined time therein (e.g. in the conduit or chamber). Further more, the device of the present invention is using high intensity pulse laser source which its associated hardware or software architecture is linked or synchronized to hydraulic or pneumatic aspects or parameters associated with the flow rate of liquids or gasses or related to the predetermined liquids or gasses types being disinfected or any combination thereof.

A novel embodiment of devices according tot the present invention wherein at least one conversion process, or plurality of processes is taking place within the laser head itself in order to maximize transmission, or minimize fiber damage thresholds or tolerances, or any combination thereof. Further more an especially useful embodiment of the present invention is having a 2nd, (or 3rd, or 4th,) harmonic generation means inside the vicinity of the laser enclosure (or head) wherein the laser in its entirety is irradiating or illuminating into (coupled) at least one optical fiber at a wavelength from about 218nm to about 1064nm and said wavelength are converted at the end of said fiber/s into an adequate wavelength or frequencies of light using at least one crystal or a plurality of crystals for irradiating or illuminating a predetermined volume of liquid or gasses therein (e.g. in the conduit or chamber) with the light.

A novel environmental embodiment of the present invention wherein the laser pumping architecture is using a) flush lamps, b) a diode pumping architecture, or any combination thereof., further more A novel enhancement to devices according to the method of the present invention wherein at least one high intensity pulsed laser source of light (A1) is aligned to a predetermined fibers matrix thread (B1) or multi track bundle of fibers (C1) or rectangular bundle of fibers (D1) or single mode arrangement of fibers (E1) or multi mode common end termination holding a predetermined volume of single strand fibers (F1) or group or mixed groups of fibers (G1) or predetermined dimensionally positioned polarization maintaining arrangement of fibers (H1) or graded index collection of fibers (I1) or plurality of gradient index fibers held together (J1) or any combination thereof (K1), and said predetermined selection of fibers are aligned to the output of the high intensity, high peak power pulse laser light source for producing plurality of pules (L1) or sequences of pulses (M1) having each high peak powers.

These variables or parameters relating to the different relationships between the flow rate of predetermined liquids or gasses (N1) through a predetermined conduit or chamber (O1) geometry or cross section or length or dimension over predetermined time (P1) and aspects of light (Q1), its associated pulsed or continuos (e.g. PW, CW) modes (R1), velocities, or energies, or timings or any combination thereof.

Further more, an especially beneficial methodology for use of devices according to the present invention is wherein the fibers end termination's are distributed and/or supported with crystals interfaces or end cups inputs/outputs which transmit light but isolates, protects and block the passage of predetermined liquids or gasses from within or throughout conduits or chambers geometry's from reaching out through the optical fibers inputs/outputs interfaces or holes or any combination thereof (e.g. Crystals interfaces, end cup, lens, beam expander, mirrors, elastic siloxan based lens, straight, bent, twisted or combination thereof) such support means suit wide varieties:
such as **a)** a ring, **b)** a rod, **c)** a straight section of metal, or **d)** plastic or **e)** polymeric compounds or **f)** rubber silicon or **g)** flat diluted rubber silicon or **h)** any conduit or chamber or **i)** The single or double or triple or any combination of opaque or reflective walls or enclosure of a disinfecting reactor (e.g. a conduit or a chamber). Further more, support means for the optical fibers integrated **j)** crystals end termination could be positioned in a predetermined parallel **k)** or sequential **l)** output path corresponding to the geometrical shape of the specific predetermined dimensions of predetermined conduit or chamber types, the direction **m)** or flow velocity **n)** of predetermined volume of liquids or gasses therein (in the conduit or chamber wherein the liquids or gasses are to be disinfected) such novel environmental embodiment comprise a variety f support and guiding means. Such means includes ; **o)** Hydraulic spiral inner extension or steering predetermined shapes or grooves or pneumatically steering wings or shaped extensions **p)** for turning and circulating the liquids or gasses therein - across the cross-section of the specific predetermined conduit or chamber for increasing their respective predetermined exposure time(e.g. slowing down flow rate by turning the liquids or gasses around (increasing friction or resistance equilibrium), (when the radiation unit is a pulse laser having high peak power per pulse **q)** A pipe or network of pipes, **r)** A grid, **s)** Network of grids, **t)** A conduit or chamber, **w)** A pond or tank/s, **x)** A mobile vehicle for intervention task forces to disaster areas suffering from floods, earth quakes, Vulcan activities **y)** A conduit or chamber for medical preparations, and/or transplants, **z)** Medical dialysis of blood and air or other related, and/or relevant liquids or gasses.

A novel embodiment of the device of the present invention wherein the same single mode fiber or fiber bundle/s used to distribute high intensity of pulsed energy (prior to performing 2^{nd}, 3^{rd}, 4^{th} harmonic generation) is simultaneously used to carry sensor data or spectroscopic data acquisition or other relevant data or machine control protocols or any combination thereof, for monitoring of illumination or irradiation or for transferring measurements of a predetermined level of suspended solids or biological compounds or non-biological compounds or turbbidity or transparency or any combination thereof associated with a predetermined volume of liquids or gases therein (inside the conduit or chamber).

A novel environmental embodiment of the present invention wherein a conduit or chamber for liquids or gases is a conduit or chamber for optical energy providing an efficient mechanism wherein liquids or gases are illuminated or irradiated or transferred simultaneously (within the same reactor geometry).

A novel environmental embodiment of the present invention wherein the crystals attached or interfaced to the end termination of the fiber or fiber bundle/s, and said predetermined bundle of fibers is coated appropriately providing sufficient electrical conductivity to change the magnetic charge of the surface of said crystal for the purpose of creating repulsion from the illuminating or irradiating (e.g. pulse or continuos modes PW, CW) or slowing or preventing the attachment of combination thereof of colloidal deposits originating or carried by hard water or air or other liquids or gases therein (within the conduit or chamber).

A novel environmental embodiment of the device of the present invention wherein at least one short laser pulse having high peak power from a high intensity source of light is more efficient over same period of time for the purpose of providing adequate disinfecting dose against noxious microorganism then average power of a CW UV lamp of the same rated power output. Further more a specially useful embodiment of the device of the present invention wherein inactivation of DNA or RNA replication sequences are the result of illumination of short pulses having high peak powers in the visible spectrum or the UV (A, B, C) spectrum or the IR spectrum or any combination thereof.

A preferred embodiment of the device of the present invention wherein at least one laser pulse lasting short duration of time from about 1 arc/sec to about 1 second and said laser pulses having high peak power from about 118mJ to about 3.18 J wherein said pulses are being projected into a conduit or chamber reactor geometry through optical fibers wherein short laser pulses having high energy are directly projected or targeted or reflected or transmitted or any combination thereof to ensure continuous (adequate) transmission of optical radiation of a specific spectral distribution over predetermined exposure time (e.g. pulse disinfecting dose or PDD), or wherein the pulses are being distributed throughout the entire length of the fiber for illuminating and/or irradiating a predetermined volume of liquids and/or gasses therein (e.g. within the conduits or chamber / reactor geometry), the ability to manipulate and deliver the light from the light source to a predetermined location within a predetermined reactor (e.g. a conduit or chamber), to split, and/or transmit, and/or diffuse, and/or deliver, and/or project and/or harmonically generate predetermined wavelengths of light or any combination thereof from a single light source, surpass current attempts, of previous methods at delivering adequate disinfecting dose without fibers. Further more previous attempts of laser delivery have not satisfied producers and/or end users of sufficient efficiencies of optical distribution and have not enter the market due to above mentioned limitation in reactor designs constrains, further more the present invention facilitate many advantages by being able to split the light from a single light source over hundreds and/or thousands of individual optical fibers, facilitating the accurate delivery of optical energy into any location within a reactor (e.g. a conduit or chamber) geometry, leaving the lamps or lasers out of the reactor vicinity, allowing the creation of important networks of optical fibers carrying, delivering and distributing high intensity of optical energy to point of use, and/or end users from a central or remotely positioned light source. The present invention facilitate an advantageous, novel, and utilitarian beneficial system geometry's, and/or architectures wherein instead of current technologies for disinfecting bacteria bringing the water to the light, the present invention takes the light into any predetermined locations through/via optical fibers-to be converted into adequate wavelength and/or frequency range for efficient illuminating and/or irradiating, or down converting, and/or harmonically generating a predetermined volume of liquids or gasses therein (e.g. in the conduit or chamber, with the fibers)

A novel environmentally preferred embodiment of the device of the present invention wherein disinfecting a predetermined volume of liquids or gasses (within the conduit or chamber) wherein the fibers are distributed or supported, or attached, or connected, or embedded. or glued, or sucked in, by reversed pressure, or flow pressure, or pressure relating to depth of liquids (e.g. water),or gasses (e.g. such as air), or solids (e.g. such as earth), or rocky layers such that are found often in oil field or petroleum wells or drilling sites.

A preferred embodiment of the present invention wherein the conduit or chamber are the filtration unit in wells for filtering suspended solids in petroleum, and/or water drillings sites on earth and in the sea where conventional currently used technologies fail to provide appropriate economically realistic solution to noxious bacteria responsible for clogging water filter grids, or matrixes, or conduits or chambers, or water cooling filter elements for drilling heads, in a predetermined drilling site, the present invention is not so limited that way the present invention could be used in a wide variety of drilling applications wherein high repetition rate, high peak power pulses from about a1) could be launched (b1) hundreds of meters under the sea (c1) or deep in the ground (d1) via or through optical fiber (e1), or bundle of fibers (f1) and wherein at their target destination light is passing through an end cup or crystal interface for harmonic generation delivering a predetermined wavelength of light into a conduit or chamber (e.g. the water filter for cooling the drill heads, deep under the ocean or ground) illuminating or irradiating a predetermined volume of liquid or gasses therein (with the light).

A preferred embodiment wherein devices according to the method of the present invention is having any combination of liquids or gasses enclosed inside natural conduit or chamber therein (e.g. inside layers of ground or rocky layers) or any combination thereof or positioned around or in predetermined proximity to said predetermined volumes of liquids or gasses in a predetermined drilling site. Such site could be under water (1), in the open sea (2), in wells for fresh water (3), or drilling for petroleum in variety of topographic or Geo.-physical locations (4). Further more, wide variety of geometry's could be explored beneficially increasing efficiency of drilling sites by providing efficient means for disinfecting bacteria or noxious microorganism which clogs important currently used filtration paths and means (5)

A novel enhancement of the devices according to the method of the present invention is having a remote high intensity, high pulse peak power laser light source (6), and said pulsed laser light source output (6a) is coupled (7) to a predetermined arrangement of single mode fiber (8), or plurality of fibers bundled (9) in a predetermined shape (9a) or packing friction (10) or any combination thereof(10a) and said predetermined fiber types are able to carry adequate intensity of pulsed energy (10b) or irradiation or illumination for irradiating or illuminating a predetermined volume (10c) of liquids or gasses therein (11) (e.g. in the conduit or chamber).

Specifically beneficial preferred embodiment of a group of devices according to the method of the present invention, is especially beneficial in drilling sites for underground search for predetermined petroleum liquid types (12) or fresh water (13), or liquids (14), or gasses (15), or any combination thereof (16) (such as drilling sites combining at least one type of liquids or gasses type which require disinfecting), further more, a specifically beneficial utilitarian enhancement to the devices according to the method of the present invention is having a remote pulsed laser source aligned to at least one optical fiber and said pulsed laser is operating in the wavelength range from about 230nm (16a), through to about 1064nm (16b), and said pulses are distributed or delivered deep under the ground (16c), through a guiding pipe (16d), or optical fiber thread pipe guide type (16e), or sea (16f), or rocky layers (16g) or any combination thereof (16h) using at least one optical fiber or bundle of fibers (16i). Such conduits or chamber (e.g. disinfecting reactors varieties) could take the shape of an elongated, or twisted, or curled, or bent, or runs in parallel or serial or combination or hydraulically or pneumatically shaped or any combination thereof for increasing or reducing flow rates of predetermined liquids or gasses or for increasing or reducing the exposure time of said liquids or gasses to said pulses (17) or continuos (18), waves of light for irradiating or illuminating a predetermined volume of liquids or gasses therein (e.g. in the conduit or chamber (wherein the fibers are distributed or supported). Such conduit or chamber could be located within the drilling head (17a) or its filtration system or it could be positioned (or integrated in to the drilling tower) before or after primary drilling has occurred or during or any combination thereof.

A preferred embodiment especially beneficial to the drilling industries of petroleum, or fresh water wherein clogging of filters in critical water (1), or petroleum (2), or liquids or gasses (2), or any combination of drilling thereof (2a), is being substantially reduced facilitating less down time , less maintenance, and less periodical replacement in contradiction to the current technologies available in the drilling industries. Further more, the method of the present invention facilitate the use of available, reasonably priced, durable, pulsed laser light sources, such as general type high repetition, high peak power lasers, known from their wide use in telecommunication industries (for data transmissions applications).

A preferred embodiment of the present invention wherein the conduit or chamber is an algae reactor having high industrial photosynthesis capabilities for CO2 fixation and/or utilization.

According to the method of the present invention is having triggered by thresholds relating to levels of the predetermined liquids or gasses flow rate or the predetermined disinfecting throughput efficiency required in according to the conduit or chamber types of geometry's used. Further more, the present invention is especially beneficial wherein the optical fiber is incorporated in, or attached to, or oriented as light conducting surface disk, such that is used in filtration units designed to stop particulate material above a predetermined size, or such that is used in back-flash cleaning operation of currently used water or air filters (e.g. filters for liquids or gasses utilizing surface disks, or membranes, or grids, or any combination thereof), such disks could have appropriate refractive index profile therein (e.g. within the disk) for appropriate transmission throughout within a predetermined conduit or chamber predetermined geometry for simultaneously filtering and illuminating or irradiating of a predetermined volume of liquids or gasses therein (e.g. in the conduit or chamber).

The present invention will be further described and illustrated in detail by figures 1-6. These detailed descriptions of the preferred embodiment do not intend to limit the scope of the present invention in any way.

Summarized List of figures for the present invention.

Figure 1 illustrate a schematic layout of fibers in a conduit

Figure 2 illustrate schematic layout of a semi holographic diffusive element in a conduit

Figure 3 illustrate a schematic layout of a combination of looped and end glowing type fiber in a chamber.

Figure 4 illustrate panoramic explosion of a multi tail optical cable assembly with a control and monitoring systems

Figure 5 illustrate a disinfecting brush (toothbrush)

Figure 6 illustrate a schematic view of a multiple types fiber orientation in a conduit

Figure 7 illustrate a schematic view of a semi holographic, partially dielectric, harmonic ring with multiple delivery architecture.

Figure 8 illustrate a schematic view of a swiveling frame with a selection of fibers and crystals for harmonic generation of optical radiation.

Figure 9 Illustrate a schematic view of a semi holographic ring featuring variable transparency conduit.

Figure 10 illustrate a schematic layout of a municipal and domestic harmonic disinfecting network.

Figure 11 illustrate a schematic view of a petroleum or fresh water well disinfecting system.

Figure 12 illustrate a schematic view of a harmonic thermally isolated and stabilized semi holographic diffusive ring.

Figure 13 illustrate a schematic view of a semi holographic, partially dielectric ring with crystal arrangement.

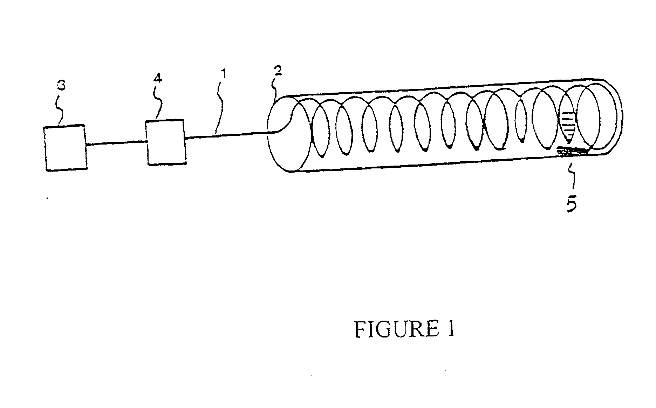

Figure 1 illustrate a schematic view of a device for disinfecting liquids or gases.

A device for disinfecting liquids or gases is shown, comprising at least one optical fiber (1) located within a conduit (2) or a chamber, and a radiation unit having a high intensity source (3) of light. The light is aligned (4) into the fibers, wherein the fiber is distributed within said conduit for illuminating a predetermined volume of liquid or gas therein. A crystal reflective member (5) is shown with at least one region of constructive interference above it.

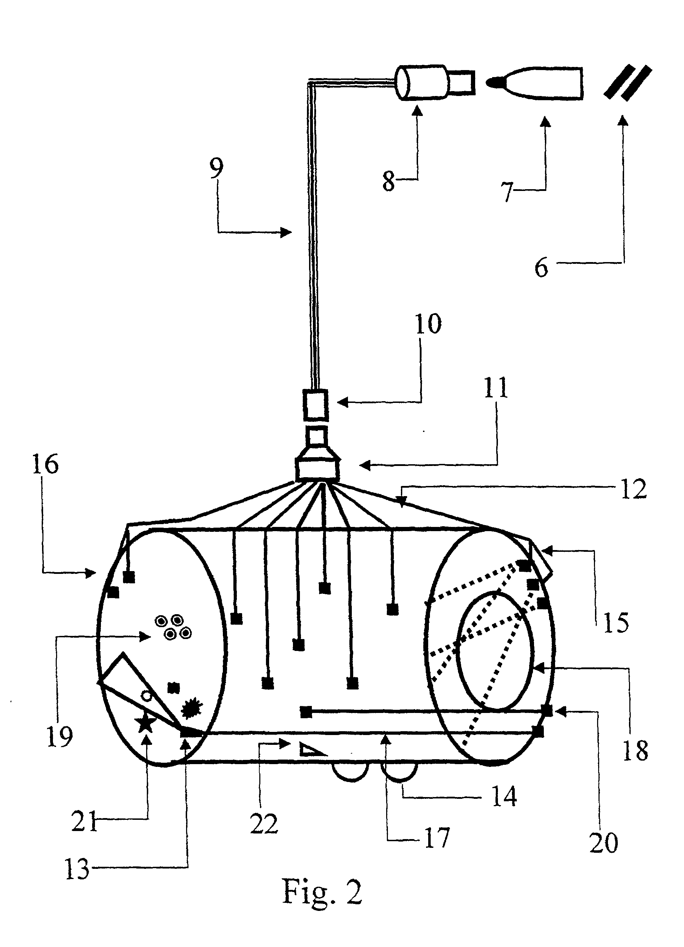

Figure 2 illustrate a panoramic view of a device for disinfecting liquids or gases. A device for disinfecting liquids or gases is shown, comprising at least one optical fiber (17) located within a conduit (14) or a chamber and a radiation unit having a high intensity source (7) of light. the light is powered by an electrical power source (6) and launches primary wavelength from 399nm to about 2800nm, aligned to a fiber harness connector (8). The light is transmitted through a light guide (9) wherein the light guide end portion (10) is connected to a central multi-tail optical fiber harness (11) from which the fibers are distributed within the conduit (14) or chamber for illuminating a pre-determined volume of liquid or gas therein (from about 180nm to about 400nm). A polarization maintaining optical fiber is illustrated on the left (16) extending to illuminate (266nm to 1064nm) the inner space of the conduit or chamber. Additional solarization resistant optical fiber (12) is illustrated on the right-extending to surround the body of the conduit marked for clarity by doted line on the left cross section of the conduit. This fiber crystal inter-mated end portion (13) is illustrated as projecting (irradiating or illuminating) harmonically generated, frequency doubled, or converted or any combinations of light beams from about 266nm to about 1064nm, the important attribution of particle size distribution (PSD) to light transmission (within the conduit or chamber) is demonstrated on the left by two particles (21) to demonstrate optimal (preferred ) condition for light transmission within the conduit or chamber - the level of Total Suspended Solids (TSS) (19) contained in a predetermined volume of liquids or gases is shown for its important attributions for light transmission efficiency (within the conduit or chamber). A crystal reflective member (22) is illustrated at the center of the conduit or chamber- reflecting or absorbing the light from the fibers sides (12) and end portions (16) on the left and (15) on the right. (20 represent a liquid light guide (in addition to optical fibers) distributed within the conduit or chamber connected at the back to the central multi-tail harness (11), a constructive interference and or diffusion (18) is shown on the right- this semi-holographic formation is originated in the radiation unit primary wavelength or frequency of light (and projected from the fibers) and its secondary (e.g. 2^{nd}, and/or 3^{rd}, and/or 4^{th}, harmonic generation, or frequency doubling, and/or converting) wave length and frequency of light in the Visible, and/or UVA, and/or UVB, and/or UVC regions or any spectral combination thereof from about 218nm to about 2400nm..

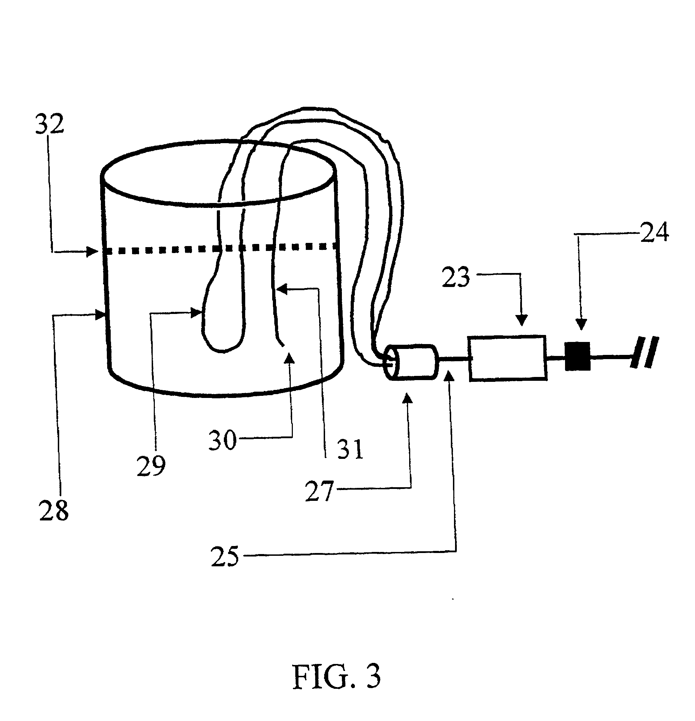

Figure 3 illustrate a schematic view of a device for disinfecting liquids or gases.

A device for disinfecting liquids or gases is shown, comprising at least one optical fiber (31) located within a conduit or a chamber (28) (could be scaled up to be a pond, a channel, container or large tanks, and or pipes, and/or sub-miniaturized for medical or surgical transplants, or in dialysis and/or in medical preparation of complex compounds, or in drinking water, or confined air). And a radiation unit having a high intensity source (23) of light wherein the radiation unit is shown here powered by electric power (24) supply unit, the primary wavelengths or frequencies of light from UVA, and/or UVB, and/or UVC, and/or Visible, and/or NIR, and/or IR regions of the spectrum or combinations from the radiation unit is aligned (27) into the fiber, wherein the fiber is distributed within the conduit or chamber for illuminating (233nm to about 532nm a predetermined volume (32) of liquid or gas therein. The distribution of fibers within said conduit or chamber are shown for clarity in both a single individual fiber distributed (31) and/or crystal terminated or intermated for direct projection and/or harmonic generation of secondary (e.g. 2^{nd} harmonic generation) out of said primary wavelength and frequencies of light. A looped distributed of side emitting optical fiber bundle (29) is shown within said conduit or chamber for illuminating and/or irradiating a predetermined volume of liquid or gas therein. e.g. with the secondary 2^{nd}. And/or 3^{rd}, and/or 4^{th} harmonically generated wavelength and/or frequencies of light.

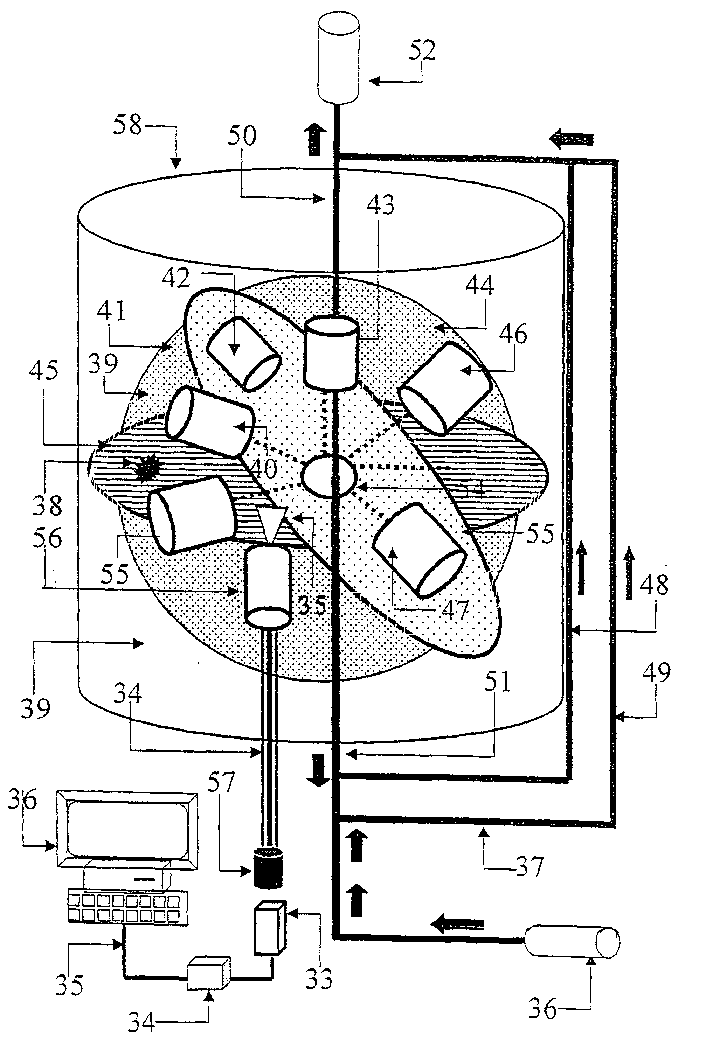

Figure 4 illustrate a schematic view of a device for disinfecting liquids or gases

A device for disinfecting liquids or gases is shown, comprising at least one optical fiber (56) located within a conduit (58) or a chamber and a radiation unit having a high intensity source (33) of light controlled by a computer (36) The light is aligned (57) into the fiber, wherein the fiber is distributed within said conduit or chamber for illuminating or irradiating a predetermined volume of liquid or gas therein. Figure 4 illustrate an integrated filter wherein the device of the present invention is illustrated as a plurality of side emitting optical fibers, and/or end glowing types and/or PM (polarization maintaining) types of optical fibers as well as fractured fibers, and/or solarization resistant fibers (56) from about 187nm to about 400nm (55) from about 249nm to about 279nm (40) from about 149nm to about 290nm (42) from about 250nm to 700nm(43) from about 180nm to about 400nm(46) from about 400nm to about 700nm (47) from 180nm to about 2400nm distributed within said conduit or chamber wherein the fibers are distributed within or around a plurality of transparent light conducting or diffusing surface disks (45)(39)(44) designed to stop particulate materials (38) in the liquids or gases disinfected (path) (51) into the filter is indicated for clarity and a recycling path marked with "R"(49 R) for the liquids or gases to pass through, pre illumination filtering module (36) and post illumination filtering module (52) are included for getting (adequate) level of particle size distribution (PSD) wherein the fibers are distributed within said conduit or chamber, for illuminating or irradiating (liquids or gases) and/or receiving light for remote feedback control of primary and/or secondary wavelength or frequencies of light. Maximizing fiber transmission efficiencies and/or damage threshold or physical or optical tolerances (e.g. using IR 1064nm as primary light to be projected direct by the fiber and/or converted up or down, and/or frequency doubled) . Using direct (e.g. end to end) fiber transmission, and/or 2^{nd}, and/or 3^{rd}, and/or 4^{th} harmonic generation (e.g. such as performed by nonlinear crystals or other means for harmonic generation ) between the radiation unit and the fibers receptive 1^{st} End termination ends, or between said radiation unit and the projective crystal or isolating lens (54, 56) 2^{nd} End termination of the fibers.

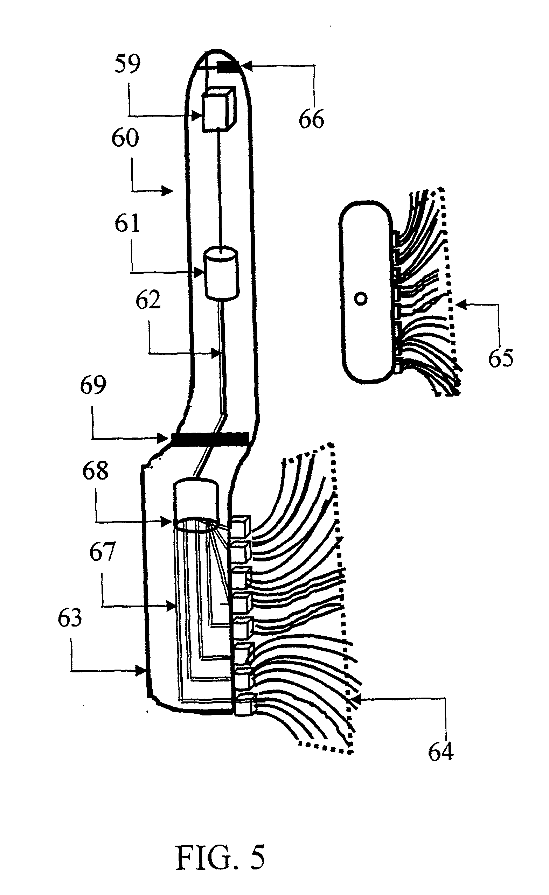

Figure 5 illustrate a schematic view of a device for disinfecting liquids or gases

A device for disinfecting liquids or gases is shown, comprising at least one side emitting optical fiber (62) located within a conduit (67) or a chamber and a radiation unit (60) having a high intensity source of primary light frequencies wherein said light is powered by batteries (59) The light is aligned into the fibers, wherein the fibers are distributed and interfacing to, or having integral or intermated crystal as reflective end cup (68) shown projecting direct, and/or 2^{nd}, and/or 3^{rd}, and/or 4^{th} harmonically generated frequencies of light within said conduit (64) or chamber for illuminating or irradiating a predetermined volume of liquid or gas therein. A spare head (65) is shown underneath the figure to indicate to the viewer that it can be replaced (e.g. together with crystal) for continuos use by dismantling or disconnecting (69).

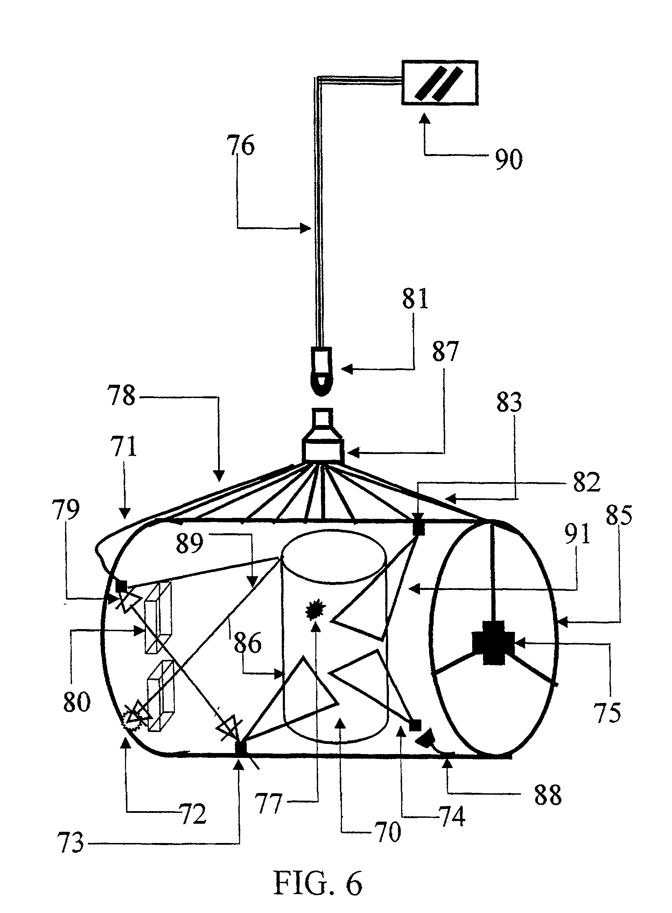

Figure 6 illustrate a panoramic view of a device for disinfecting liquids or gases.

A device for disinfecting liquids or gases is shown comprising at least one region of constructive interference (70) generated from the holographic element (80) which is attached to the frame (enclosure) of a conduit (85) or a chamber aiding in the elimination of head loss within the conduit or chamber) positioned opposite a reflective
member (on the top left hand side) (79), bottom left hand side (72) top left (82), bottom right (74), top (89), top (82), solid particle (77) is illustrated for its important attribution to light transmission within the liquids or gases to be disinfected,
a radiation unit having a high intensity source (81) of (coherent) light from about 200nm to about 700nm, the radiation unit is powered by electrical power supply unit (90), coherent light from the radiation unit is aligned (87) to at least one (side emitting) optical fiber (left top) (71), bottom right (88) surrounding the body of the conduit (85) or chamber, (75) represent a secured opening on the far right hand side (a secured lid or cover) Figure 6 illustrate a conduit or a chamber wherein (a hologram is formed) out of at least one region of constructive interference created by light beams (of axis or reference beams) (91) Figure 6 illustrate coherent light from the radiation unit (81) is aligned to a multi tail harness (87) the light gets carried by fiber (71) to a reflectance member (79) and gets projected through the holographic element (80) positioned directly in front of it (in front of the reflective member) at least one side emitting optical fiber is distributed within the conduit or chamber for illuminating (91) (86) or irradiating a predetermined volume of liquid or gas therein.

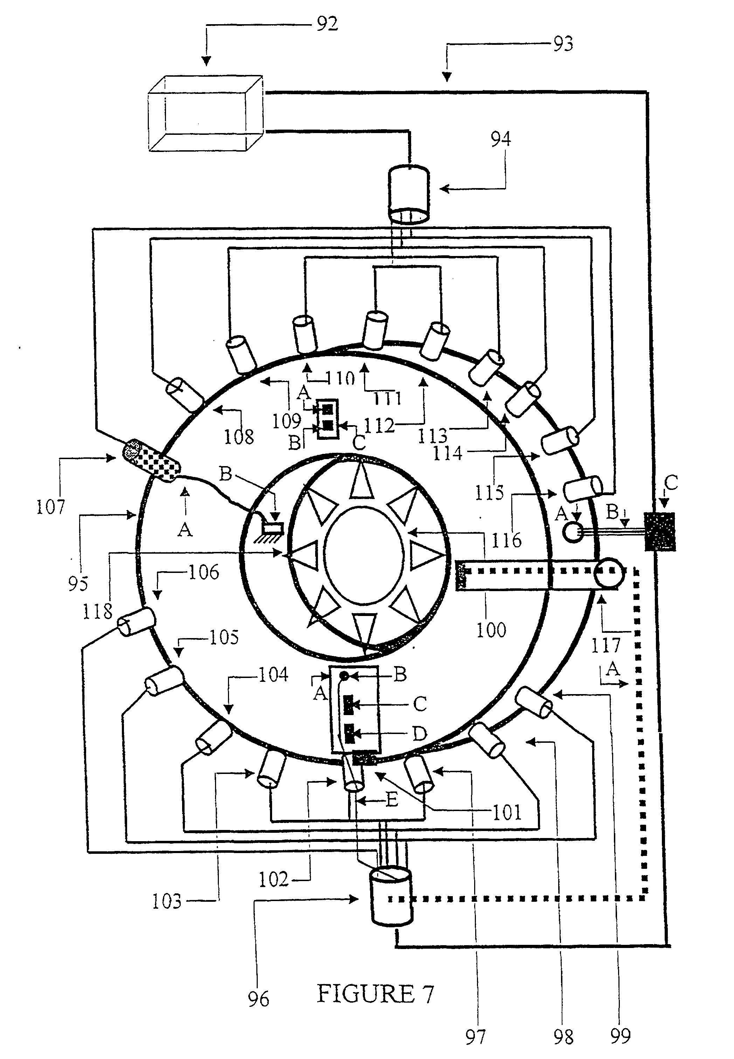

Figure 7 illustrate a panoramic view of the device for disinfecting liquids and gasses A device for disinfecting liquids and gasses is shown, comprising; Distributing at least one optical fiber (93), in the region (118) to be disinfected; Aligning at least one radiation unit (92) having a high intensity source of primary light from about 218nm to about 1064nm into said fibers; Radiating said liquid or gasses by said optical fiber over a predetermined period of time. (94) represent a multi- fiber assembly splitting the single beam to 10 individual split independent feeds (107, to 116) enhancing disinfecting reactor geometry's for producers and/or end users alike which currently do not have efficient means to split optical radiation for the purpose of disinfecting liquids and gasses which the present invention not being so limited can be used for splitting and transmitting and radiating and/or delivering or diffusing, or projecting from said fibers) "unlike previous methods which bring the water (e.g. such as liquids and/or gasses) to the light" the present invention bring the light to the liquids and gasses each feed is reaching the ring through fibers, delivering primary wavelength (e.g. such as 1064nm in the IR region and/or 266nm in the UVB region), by providing a semi holographic (100), (118), diffusive, partially dielectric ring support means (95) from the radiation unit into 10 of the ring's inputs, (into the ring) and/or outputs (107-116) e.g. into and/or out of a conduit or a chamber (which for the purpose of the present invention could be transparent, or translucent, or semi opaque having beneficial diffusive properties for diffusing optical radiation and or adequate refractive index profile for conducting light throughout (e.g. guiding light by total internal reflection and liquids and gasses hydraulically by pressure simultaneously) for the purpose of disinfecting projected from outside said transparent conduit or chamber) wherein the fibers are distributed. And said fibers are used in bi-directional transfer mode wherein each (108), (109), (110), (111), (112), (113), (114), (115), (116), of the individual fibers bundles is reaching substantially to a predetermined distance from the radiation unit and terminating, and/or inter-mating to at least one thermally isolated and/or stabilized modular harmonizing crystal interface (e.g. such as KTP and/or PPKTP and/or LBO crystals, or equivalent electro-optical conversion means for generating 2^{nd}, and/or 3^{rd}, and/or 4^{th}, and/or 5^{th} harmonics). A plurality of non linear crystals selected and sequentially positioned (108-116) e.g. inside a plurality (not shown) of wavelength specific crystal equipped cartridges (101) wherein said cartridge containing the crystals is thermally isolated (C), having temperature stabilization sensing means (A), additional optical fiber (B), is shown for clarity interconnecting to the same network extension feed or multi tail fiber assembly carrying signals to controller (not shown) (96), (94). (e.g. such as multi - tail fiber harnesses). The crystals are represented for clarity by (D) and (E), secured at a predetermined angle or phase matched at a predetermined distance from the sensing means (A), The ring's I/Os, (input/outputs) are set to receive primary optical radiation from the radiation unit wherein the fibers are aligned to the radiation unit. The optical fibers or interfaced, or inter-mated or integrated (102), (103), (104), (105), (106), to additional fiber inputs leading to additional cartridges (not shown) wherein the optical radiation is delivered unchanged (e.g. no wavelength change) for direct spectral transmission from the radiation unit. So the same ring support means deliver optical radiation into the liquids or gasses flowing throughout its optically covered (100), (118) inner dimension. A plurality of wavelengths (e.g. both direct or harmonically generated or frequency doubled such as SHG, THG, FHG) are being delivered by the distribution of fibers for radiating a predetermined volume of liquids and gasses therein (with the light from the fibers). (107) is shown for clarity as having a polydimethil(phenyl)siloxan based transparent diffusive tips grouped together (107 B) in a shape of a brush and extending substantially into the liquids and gasses to be disinfected. The brush is optically interfaced to a diode type laser (e.g. or bar of diodes) positioned sequentially, substituting fiber input (107) (shown her in dark color for clarity) and excepting its primary pumping frequency from the radiation unit (92) from about 400nm to about 670nm via optical fiber feed shown extending from fiber assembly (94) and reaching into said diode laser. (A) represent a shorter segment of fiber feeding radiation to the siloxan based brush tips. Adequate contact and/or exposure with the liquids and gasses passing through the ring is maximized. The diffusive tips further reduce headeloss and help maximizing effective inactivation of noxious microorganism or noxious bacteria and/or microorganism in the liquids and gasses to be disinfected. (116 A) is shown on the right illustrating a temperature isolation and stabilization sensing means, connected through (B) to the radiation unit (94), an optical junction is shown (C) interconnecting and encapsulating both; the main multi tail optical cable assembly (93) and sensor connecting fiber (B) together for easy integration to wide variety of environments or site topographies (117) is shown as empty fiber insert point for taking optical radiation via optical fibers to a remote control and data acquisition unit (not shown) for feedback control of relevant parameters of the system (117 A) is showing using a doted line the path of additional fiber to be connected into fiber insert (fiber not shown).(110) is representing a crystal cartridge having two individual crystals in it (B), (A), (the crystals are aligned and phase - matched to the fiber 2^{nd} End termination (not shown) and are thermally stabilized and or isolated.

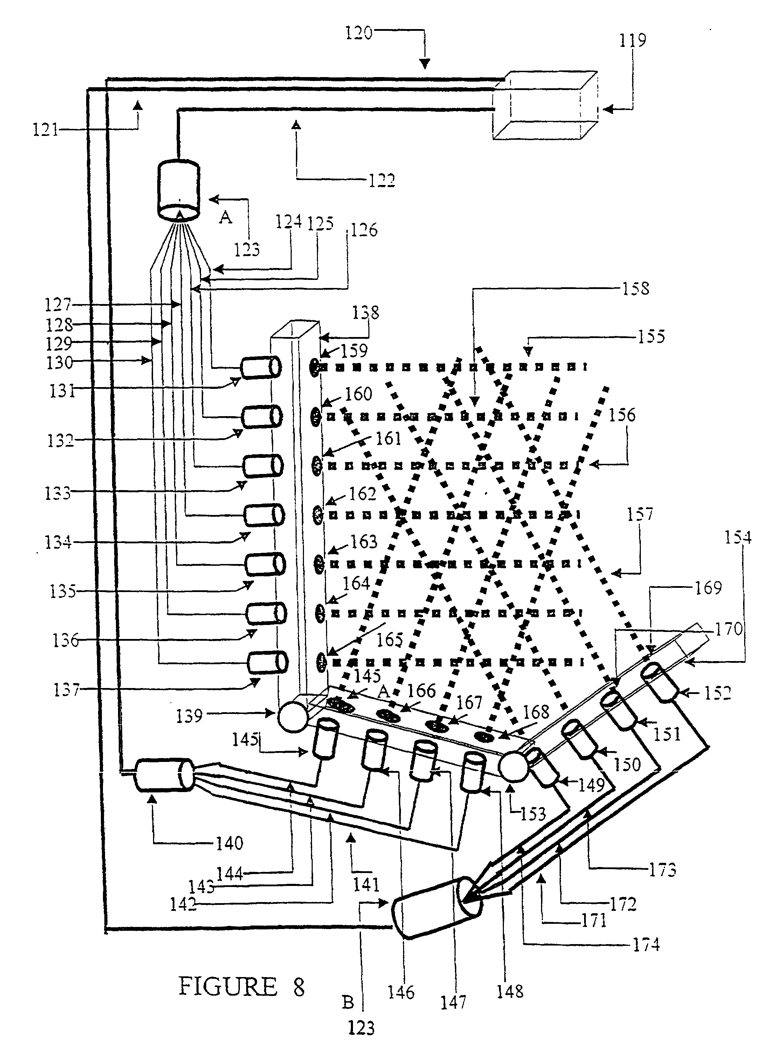

Figure 8 illustrate a schematic view of the device for disinfecting liquids or gasses. A device for disinfecting liquids and gasses is shown, comprising; Distributing at least one optical fiber (120), (124), (125), (126), (127), (128), (129), (130),(142), (143), (144), (171), (172), (173), (174), or bundle of fibers (122), (121), in the region to be disinfected (158), (156), (155), (157); Aligning at least one radiation source having high intensity source of fight (119), into said fibers (alignment not shown); Radiating said liquids and gasses by said fibers over a predetermined period of time. (131 to 137) represent fiber inputs each having means for transmitting through identical or down converted optical radiation at a predetermined wavelength and frequency. (139), and (153) illustrate pins allowing the entire frame to adjust to wide variety of geometry's.(159 to 165) represent elastic or durable lenses each having a predetermined divergence as so collectively filling the inner space of the frame (154), additional transparent elastomer based lenses (e.g. such as polydimethylmethilsiloxan) are illustrated (145), (166), (167), (168) having a different divergence or focus for the purpose of radiating a predetermined volume of liquids and gasses therein (e.g. throughout the frame). Fiber inputs (151), (152), (150), (149), (153), are illustrated delivering optical radiation from about 180nm to about 2600nm wherein primary wavelength from the radiation unit is converted at least once into SHG, and/or THG, and/or FHG, and/or any spectral combination thereof. (e.g. 2^{nd}, 3^{rd}, 4^{th}, harmonic generation using thermally isolated or stabilized crystal cartridges (not shown). (138) illustrates additional swiveling frame support means.(146), (147), (148) illustrate individual fiber feeds (142-144) extending from fiber assembly (140), wherein primary wavelength from about 140nm to about 2600nm is being delivered and/or converted and/or diffused into the liquids and gasses therein (e.g. throughout the frame).

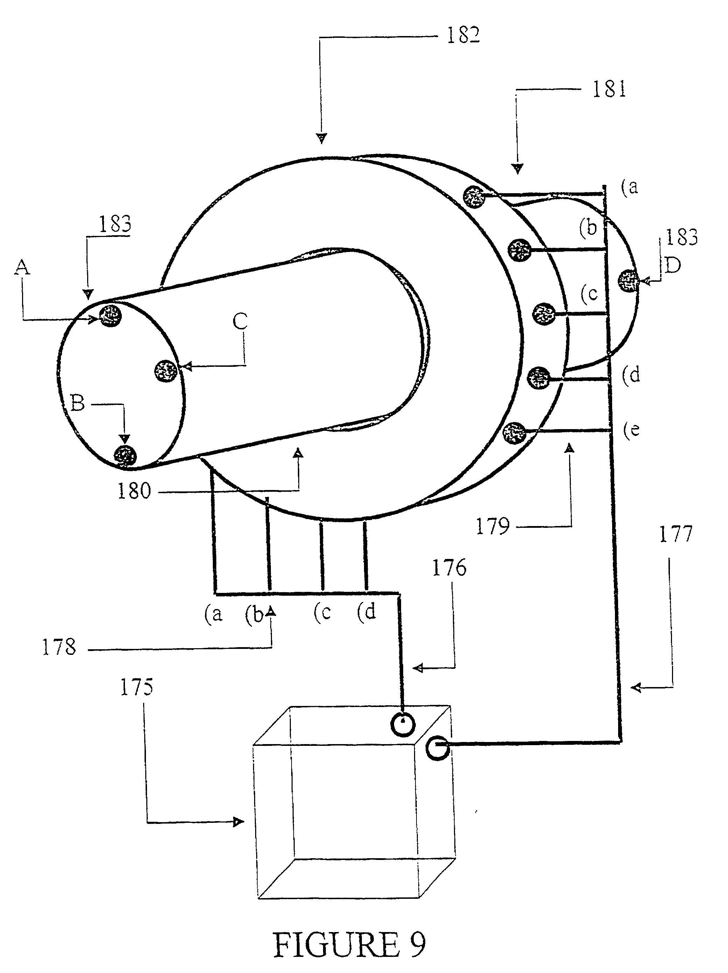

**Figure 9** illustrate a schematic view of the device for disinfecting liquids or gasses. A device for disinfecting liquids or gasses is shown comprising; Distributing at least one optical fiber (181), (179), (176), (177 a, b, c, d, e,), (178 a, b, c, d,), in the region to be disinfected. Aligning at least one radiation unit having a high intensity source of light (175), into said fibers; Radiating said liquids and gasses by said optical fibers over a predetermined period of time. A semi holographic, partially dielectric, thermally isolated or stabilized ring (182), coupled (e.g. by quick coupling) to form a continuing conduit (the conduit could be transparent, or opaque, or translucent, or have a predetermined refractive index profile therein for guiding light in accordance with conditions for total internal reflections) at each side (183) and (183 a, b, c) illustrates sequentially positioned fiber inputs (a, b, c) wherein (183 d) is positioned at the other side of the ring for alternating the delivery of optical radiation of a specific spectral distribution between cross section radiation from the ring (182), and the longitudinal projection points (183 a, b, c,) of the fiber inputs (183 a, b, c, d) parameters associated with the liquids or gasses to be disinfected (e.g. such as turbidity, or transparency or levels of suspended solids of water or air) provide thresholds for feedback control and/or triggering of the radiation unit and/or control unit (not shown).

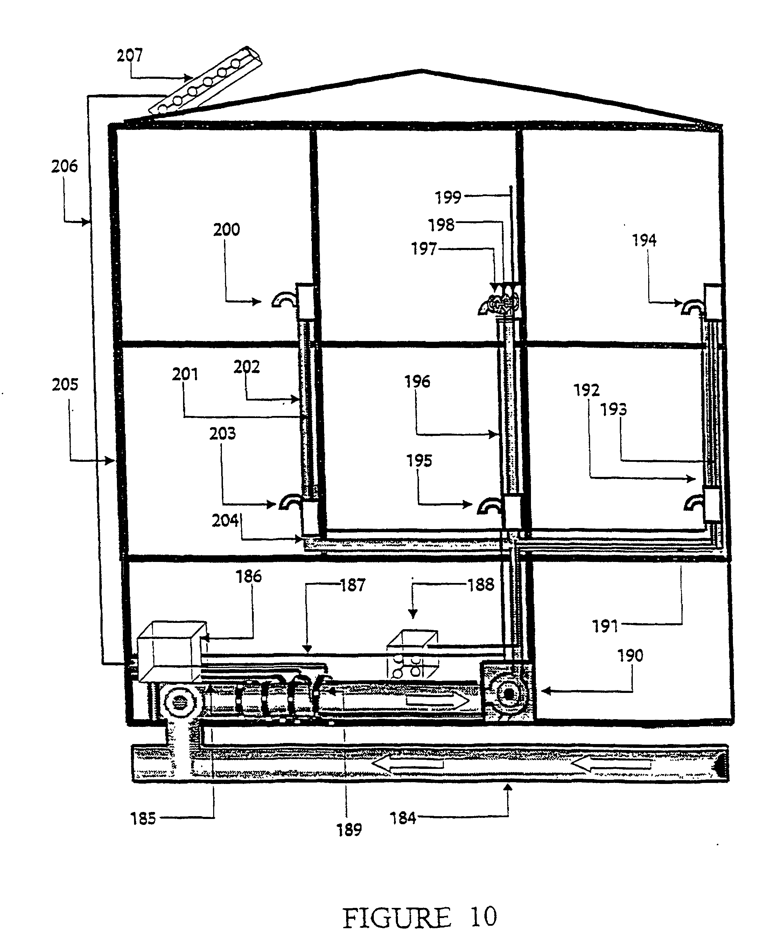

**Figure 10** illustrates a schematic network view of the device for disinfecting liquids or gasses. A device for disinfecting liquids and gasses is shown comprising; Distributing at least one optical fiber (185), (187), (191), (193), (201) in the region containing the liquids and gasses to be disinfected; Aligning at least one radiation unit having a high intensity source of light (186), into said fibers; Radiating said liquids and gasses by said optical fiber over a predetermined period of time. An interactive optical infrastructure network is illustrated for disinfecting liquids and gasses comprising; Distributing (in a conduit or chamber) in the region of the liquids and gasses to be disinfected at least one optical fiber (196), 191), having receptive 1^{st} End termination, and 2^{nd} End extending substantially to reach into the conduit or chamber, and inter-mated on entry at a predetermined angle, to a at least one modular 2nd, and/or 3^{rd}, and/or 4^{th}, harmonic generation crystal module interface (197), (198), (199), (189), wherein the crystal is terminated with an isolating transparent lens (not shown) or diffusing tips (not shown) facing the liquids and gasses to be disinfected; Aligning at least one radiation unit (186), having a high intensity source of light into said fibers; Launching through receptive vacuum, or thermally protective interface (188) into said fibers 1st En^{d} termination (not shown), high intensity light having primary wavelength in the Visible, and/or NIR, and/or IR, and/or UV (UVA, UVB, UVC) regions of the spectrum; Illuminating or irradiating (199, 198, 197,) a predetermined volume of liquids and gasses with the delivered light having identical, or secondary, or tertiary wavelength range in the UVA, and/or UVB, and/or UVC, and or Visible, and/or IR regions of the electromagnetic spectrum or any harmonically generated, or converted spectral combination thereof over a predetermined period of time. (204) illustrates infrastructure support means (204), indicating for clarity that the fibers can reach substantially to cover large area (unlike previous methods and means) e.g. using a single light source. (203), (200), (195), (194), (192), represent end users point of use (e.g. such as a tap, or network of pipe extensions). (190) Illustrates a central pump (with arrow facing the pump for clear indication of the liquids and gasses direction of flow) for distributing liquids and gasses throughout the house or building (205), which arrive from lower main liquids and gasses supply line (marked with two arrows pointing left). Rooms in a house on different floors is herewith illustrated to further illustrate splitting and delivery (covering large areas) or diffusion by optical fiber network reaching substantial distances).A solar panel is illustrated (207) on the roof of the building or house powering the radiation unit (185).

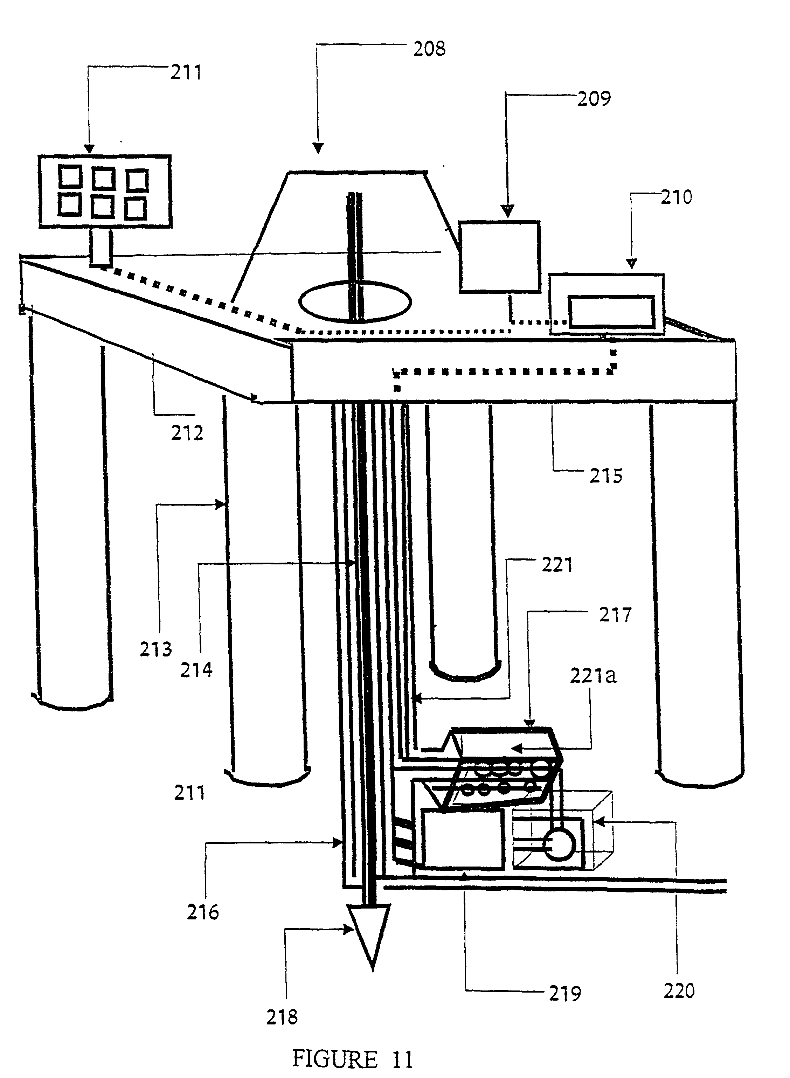

**Figure 11** Illustrates a device for disinfecting liquids and gasses. A device for disinfecting liquids and gasses in wells and drilling sites is forthwith shown, comprising; Distributing at least one optical fiber (215), (221), (216), (214), in the region containing the liquids or gasses to be disinfected; Aligning at least one radiation unit (210), having a high intensity source of light into said fibers; Radiating said liquids or gasses by said optical fibers over a predetermined period of time. Figure 11 illustrates the ability of the device of the present invention to disinfect liquids or gasses (e.g. such that are found in petroleum and/or fresh water drilling sites and wells, both, under the ocean and under the ground) in drilling installation requiring solution to clogging (clogging of water filters for cooling systems to the drilling heads and/or water from the earth which have being contaminated) The present invention by delivering radiation using optical fibers can provide easy solution to clogging enhancing productivity and saving down time and expenditure caused by maintenance and/or expensive periodical replacement procedures.

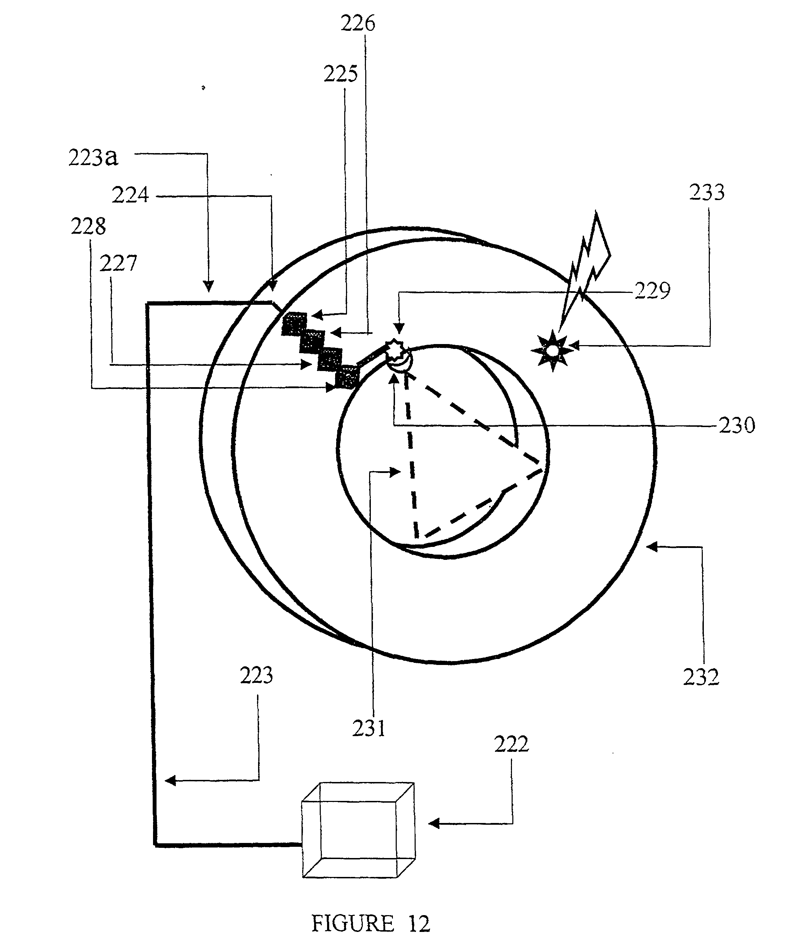

**Figure 12** Illustrates a device for disinfecting liquids or gasses. A device for disinfecting liquids or gasses is shown, comprising; Distributing at least one optical fiber (223), (223a), (224), in the region containing the liquids or gasses to be disinfected; Aligning at least one radiation source (222), having a high intensity source of light into said fibers; Radiating said liquids or gasses by said optical fibers over a predetermined period of time. (225), (226), (228), (227), represent a crystal arrangement (e.g. a cartridge) through which primary wavelength light from the radiation source from about 160nm to about 2600nm gets transmitted, and/or identically delivered, and/or harmonically generated or frequency doubled, and/or up or down converted according to species specific calibration standards for disinfecting using the device of the present invention. A transparent elastomeric lens (230), is being illustrated as isolating the liquids or gasses from the inner surface of the crystals (e.g. cartridge not shown). The crystals are thermally isolated and/or stabilized (not shown) in a cartridge. The crystals are arranged in a semi holographic, dielectric ring (230), wherein the fibers reach and are to be inserted into the ring (one fiber is shown to reach the ring (223) delivering optical radiation of a predetermined spectral distribution. (233), illustrates the divergence of the isolating lens wherein the beams coming out of the lens (e.g. after passing through the crystal) identical to the primary wavelength and frequency of light from the radiation unit (222) or the light from the radiation unit could be converted into SHG, THG, FHG (E.G. 2^{ND}, and/or 3^{rd}, and/or 4^{th} harmonic generation and/or frequency doubling) converting said primary wavelength from about 1064nm to about 532nm, or from 532nm to about 266nm (depending on the choice of crystals in the cartridge (not shown) light is shown at a predetermined divergence (231) filling the inner space of the ring. An electric pulsed DC/AC input to the partially dielectric ring is shown for clarity by (233) lowering the impact of colloidal deposits and/or hard water deposits by contradicting the polarity and/or magnetic field of organic suspended solids in the liquids or gasses to be disinfected.

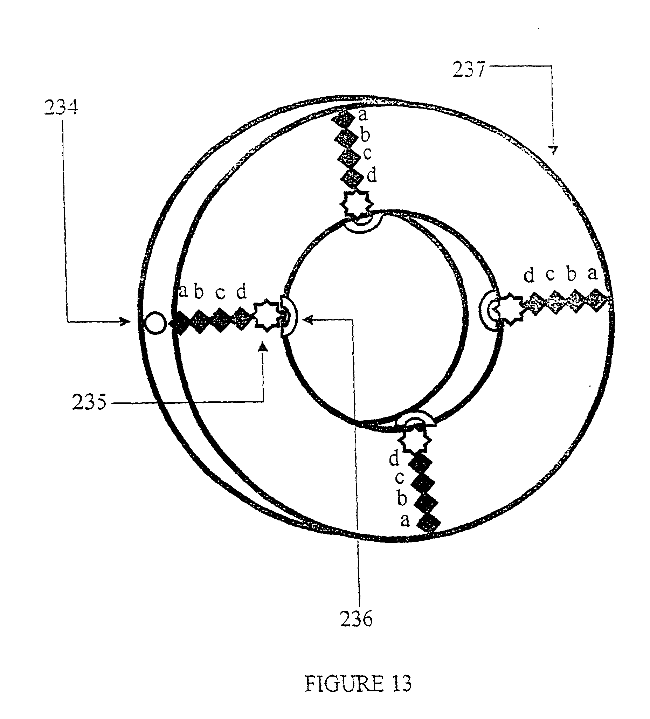

**Figure 13** illustrates a device for disinfecting liquids or gasses. A device for disinfecting liquids or gasses is shown, comprising; Distributing at least one optical fiber (not shown) in the region containing the liquids or gasses to be disinfected (one optical fiber input is illustrated (234); Aligning at least one radiation unit (not shown) having a high intensity source of light into said fibers; Radiating said liquids or gasses by said optical fibers over a predetermined period of time. The crystals (a), (b), (c), (d) are arranged in a thermally stabilized or isolated cartridge (not shown). The crystals are receiving optical radiation from the radiation unit at a primary wavelength and frequency from about 200nm to about 2400nm wherein the crystals (e.g. such as a KTP and/or PPKTP type or equivalent) convert said primary wavelength to a lower wavelength (e.g. such as a 532nm and/or 266nm) in the Visible and/or UVA, UVB, UVC region of the spectrum. A transparent lens is being illustrated (236) isolating the liquids or gasses to be disinfected from the crystal cartridge. The ring is also thermally stabilized or isolated for maintaining and/or maximizing the conversion efficiency of the crystals.

### Experimental

The experiment was held at the department of Physical Chemistry laboratories at the Hebrew University in Jerusalem. Sigma Chemicals Israel Company have supplied 20 liter of the E-Coli fermented culture. Biological solutions and patric dishes were prepared.

Each stage ofthe experiment included two separate modes of Electro-magnetic radiation
a. Pulse laser at a repetition rate of 10Hz. Wavelength rated 93% at 266nm and 7% at 532 nm.
b. Polychromatic continuous wave UV lamp with a broad bend (100 nm) centered at 300nm

### Equipment:

Laser setup - Nd/yag laser - Spectra Physics - Quanta ray model Nd/yag DCR with Spectra A-702 2nd and 4th harmonic generation modules, computer controlled, having wavelength of laser radiation measured at 266nm (93% at 266nm, 7% at 532nm), Pulse duration: 10ns Pulse powers were measured as : Fiber delivery runs: measured 4mJ per pulse at the end of the fiber, direct irradiation (without the optical fiber) measured at 70mJ (before entering the liquid) .

### Optical signal path:

(a) Laser head,(b) 1^{st} steering mirror, (c) 2nd steering mirror, (d) 3rd steering mirror, (e) 1st focusing lens, Oriel FO-315, (f) 2nd collimating lens, Oriel CO-318, (g) Directive prism (90 degrees at 80% efficiency) (e) fiber.

### Measuring devices:

"Offir Optronics" power meter, Scientec model 364 - laser power meter (1mj-2joule)

The fiber types used throughout the experiment: HGFS (high-grade fused silica) optical fiber bundle (manufactured by Oriel), Bi-furcated, 10 mm total physical diameter for each arm, (2.5 -3mm NOD) HGFS total Bi-furcated bundle Length 1 Meter, bundle having a PVC/polyamide type sheathing opaque, metallic strength member.

A SiO2 (Homogenize Glass) fiber bundle, Atlantium type, at 8mm diameter comprising 5000 individual 50-micron fiber strands. Flat transmission at UVA spectrum. Total length of bundle 3-Meter (originally a loop of 1.5 Meter out, 1.5 Meter in) fiber N/As is less then 0.7. nominal. No sheathing.

### The components in the biological set up are:

Stainless steel sealed container, Ø170 mm, pierced with two fibers bundles for irradiation protection. SiO2 (crown glass) 1 liter Ø100 mm (large diameter dish). Magnetic stirrer base, Wild type of E.coli K12 strain 20 liter (for adequate sampling), Patric dishes with Seline (biological solution) for seeding.

### The Experiment setup:

The laser head was positioned 5 meters from irradiation target (Wild type of E.coli in 1 liter of water). The lenses to steer and direct the laser pulsed beam across the room were located above a tabletop surface. Mounting devices were used for holding a prism to direct the light beam down at a 90-degree angle.

The fiber bundles were aligned to the laser and connected in front of the prism preceding dual lens configuration for minimizing losses.

Surface losses in the optical chain: 1^{st}, 2^{nd}, 3^{rd} steering mirrors; 1^{st}, 2^{nd} focusing lenses; prism surfaces loss; Each pulsing surface loses approx. 4% of total optical power output available at the next surface totaling a surface loss of approx. 27% of output. Radiation through a distance of 6 meters of air should count for additional 3%

Polychromatic lamp setup - Oriel UV Xenon lamp XU-450 rated Power 450W, reduced by filters, lenses, attachments. (50mW CW) measured at each end of HGFS bi-furcated fiber bundle. Attached filters were used for separating deep UV radiation at output. The lenses used: Collimator lenses , Paralleling beam lenses with spectral range 60% of irradiation at UV-VIS, enhanced at between 220nm through to 325nm respectively, a filter 7-54 Centered at 300nm

### Experiment description

The Laser phase of the experiment included three modes of irradiation:-
**a)** LD (large dish)/Loop ( optical fiber where both side of the loop are connected into one end termination) - Irradiation / radiation using SiO2 side emitting fiber bundles (5000 50 micron fibers) aligned into the laser beam. One meter of the bundle was stripped of its sheathing and distributed in the experiment dish. The dish was exposed to external light during the irradiation.
**b)** LD/O.F - Irradiation/ radiation into HGFS, end-glow, bi-furcated fiber bundle 1 meter long aligned into the laser beam. The end-glows were inserted through angle-adjustable holes in the stainless steel container cover. The container was sealed during the irradiation.
**c)** LD/Direct - the UV was irradiated / radiated directly (not through the optical fibers) into the dish via the prism.

The UV CW lamp phase - a Xenon lamp 450-Watt nominal UV - the irradiation was channeled into a HGFS, end-glowing, bi-furcated fiber bundle 1 meter long aligned into the laser beam. The end-glows were inserted through angle-adjustable holes in the stainless steel container cover. The container was sealed during the irradiation.

In both phases - exposure time was 600sec (10 minutes) wherein periodical samples were extracted using pipette driven disposable tips. A solution was prepared for counting the culture during the next 24 hours. Seeding 14:00 20/7/98, counting 14:00 21/7/98.

Summary - POC results (e.g. proof of concept experiment results

Counting of developed cultures of E.coli. Wild type, 24 hours later. The results were summarized in the following table:

| EXPOSURE DURATION | PULSE LASER IRRADIATION | | | LAMP |
|---|---|---|---|---|
| | LD/Loop (a) | LD/O.F (b) | LD/Direct(c) | LD/O.F |
| 2 Seconds | 0 | 0 | 12 | 38 |
| 10 Seconds | 0 | 0 | 2 | 14 |
| 30 Seconds | 0 | 0 | 1 | 23 |
| 60 Seconds | 0 | 0 | 0 | 7 |
| 180Seconds | 0 | 0 | 0 | |
| 300Seconds | 0 | 0 | 0 | |
| 400Seconds | | | | 7 |
| 600 Seconds | 0 | 0 | 0 | 4 |
| REFERENCE (24 hours - no irradiation) | 49 | 42 | 47 | 45 |

The results as shown in figure 1 indicate the very fast disinfection (less than 2 seconds) was achieved by using the side emitting fiber (mode a) and end-glow fiber (mode b), direct irradiation / radiation (mode c) showed total disinfection after 1 minute. Reference results using UV lamp through fiber appeared to show much slower disinfection (total disinfection was not achieved). The UV lamp results correspond to the known characteristics in existing literature.

Contrary to the existing convention, frequently backed by researchers in relevant environmental fields and contrary to the existing knowledge, which is available in the public domain, it is not the sheer number of photons absorbed into the links of DNA and RNA which effect and deactivate replication sequences. It can be performed using a short but high-energy pulse with 4 times less photons then the quantity present in a medium power continuous lamp (e.g. the CW type UV lamp used in the experiments).

Appropriate statistical sampling and counting was done. No exemptions were observed.

## Claims

1. A method for remote disinfecting liquids and gasses comprising; distributing at least one optical fiber in the region containing the liquids or gasses to be disinfected; aligning at least one radiation unit having a high intensity source of light into said fibers and radiating said liquid or gasses by said optical fiber over a predetermined period of time, wherein said radiation unit is a laser and wherein the primary radiation frequency of the laser is converted to another secondary frequency, before or at the disinfecting site, such that the secondary radiation emitted from the optical fibers has a different frequency from the primary laser frequency.

2. A method according to claim 1 wherein the conversion of primary frequency into secondary frequency is harmonically generated.

3. A method according to claim 1 wherein the optical fibers are integrated into the body or walls of a conduit or chamber containing the liquids or gasses to be disinfected.

4. A method according to claim 1 having at least one side emitting optical fiber distributed in the region containing the liquid or gasses to be disinfected.

5. A method according to claim 1 having at least one end glow optical fiber distributed in the region containing the liquid or gasses to be disinfected.

6. A method according to claim 1, further transferring in real time spectroscopic data by optical fibers from the disinfected region to any remote location for feedback control of the disinfecting process.

7. A method according to claim I wherein the radiation unit has a single wavelength monochromatic high intensity light source.

8. A method according to claim 1 wherein the radiation unit is a polychromatic high intensity light source from about 249nm to about 2400nm

9. A method according to claim 1 wherein the radiation source is a pulse high intensity source of light.

10. A method according to claim 9 wherein the pulses from the high intensity source of light are aligned to optical fibers selected from: Single mode, multi mode, graded index, gradient index, or polarization maintaining fibers or bundles of fibers.

11. A method according to claim 1 wherein the radiation unit is a continuos high intensity light source

12. A method according to claim 1 wherein the radiation unit having a high intensity Ultra Violet light from about 187nm to about 400nm.

13. A method according to claim 1 wherein the radiation unit having a high intensity source of visible light from about 400nm to about 700nm.

14. A method claim 1 wherein the radiation unit have a high intensity source of IR from about 800nm to about 2400nm.

15. A method for furnishing a network with interactive optical infrastructure for disinfecting liquids or gases, comprising; distributing in a conduit or a chamber containing the liquid or gas to be disinfected, at least one optical fiber having receptive 1st end termination, and 2nd end extending substantially to reach into the conduit or chamber, and inter-mated on entry at a predetermined angle, to at least one modular 2nd, and/or 3rd, and/or 4th harmonic generation crystal module interface, wherein the crystal is terminated with an isolating transparent lens facing the liquid or gasses to be disinfected; aligning at least one radiation unit having a high intensity source of light into said fibers ; launching direct, and/or through receptive vacuum interface into said fibers 1st End termination, a high intensity light having primary wavelength in the Visible, and/or NIR, and/or IR and/or UV regions of the electromagnetic spectrum; illuminating or irradiating a predetermined volume of liquids or gasses with the delivered light having a secondary wavelength range in the UVA, and/or UVB, and/or UVC, and or Visible, and/or IR regions or any harmonically generated, or converted spectral combination thereof over a predetermined period of time.

16. A device for disinfecting liquids and gasses by the method as defined in claim 1, comprising a conduit or a chamber containing the liquids or gasses to be disinfected, at least one optical fiber distributed within or around or integrated into the walls or the body or the vicinity of said conduit or chamber, and a laser having high intensity source of light aligned into said fibers wherein at least one crystal interface is attached or integrated to at least one optical fiber or bundle of fibers end termination for harmonically converting the wavelength of the incoming primary pulse to a lower wavelength of outgoing pulse.

17. A device according to claim 16 wherein the laser is a polychromatic or monochromatic light source from 220nm to about 2400nm.

18. A device according to claim 16 wherein the laser is selected from flash lamp, fiber laser, solid state laser, gas laser, crystal laser.

19. A device according to claim 16 wherein the fibers are aligned of interfaced into the radiation unit through a vacuum flange or collimating optical interface increasing the damage threshold of the fibers, allowing the delivery of substantial optical energy of a specific spectral distribution.

20. A device according to claim 16 especially useful for disturbing the breeding cycle of cockroaches wherein the light emitted from the fibers is in the visible region of the spectrum.

21. A device according to claim 17 wherein the radiation source is a polychromatic microwave excitation lamp.

22. A device according claim 17 wherein the radiation unit is substituted by a solar collector and/or concentrator harnessing global solar radiation.

23. A device according to claim 16 having at least one side emitting optical fiber.

24. A device according to claim 16 having at least one end glow optical fiber.

25. A device according to claim 16 having means for supporting the fibers within the conduit or chamber.

26. A device according to claim 16 having a transparent or opaque sleeve enclosing the optical fibers.

27. A device according to claim 26 wherein the sleeve is integrally enclosing the optical fibers.

28. A device according to claim 16 wherein the optical fibers in the conduit or chamber is geometrically distributed for the production of at least one region of constructive interference in the conduit or the chamber.

29. A device according to claim 16 wherein the fibers are side emitting fibers, distributed geometrically in a spiral or zigzag for the forming of a plane, a cone, a cylinder, or a smooth surface or combination thereof.

30. A device according to claim 16 wherein the fiber is bent back along its own path to form at least one section of parallel fiber path.

31. A device according to claim 16 having a reflective member parallel to at least one radiating section of the distributed fiber.

32. A device according to claim 16 having a reflective end cup affixed to the terminal end of the optical fiber for the production of at least one region of constructive interference in the conduit, or chamber or any other predetermined space.

33. A device according to claim 31 wherein the fiber is a side emitting fiber and wherein the reflective member is an integral part of the fiber.

34. A device according to claim 17 wherein the fiber is an end glowing type of fiber, and wherein fibers are dimensionally arranged for the production of at least one region of constructive interference.

35. A device according to claim 17 having in addition a holographic optical element for the production of at least one region of constructive interference in the conduit, or a chamber, or any other predetermined space.

36. A device according to claim 16 having an ultra violet hologram in the conduit or chamber, and said hologram is formed from at least one constructive interference and said hologram is filling most of the conduit or chamber inner space.

37. A device according to claim 17 wherein the conduit or chamber have a filtration unit having at least one porous screen, or membrane, or surface disk, or capillaries, or magnetic elements or compounds for the removal of particulate materials and wherein the optical fiber5s are integrated into or onto at least one of the screens, or membranes, or surface disk of the filtration unit.

38. A device according to claim 16 wherein the conduit or chamber is sewage pipe.

39. A device according to claim 16 wherein the chamber is a closed space selected from the aeration volumes of loosely packed soil, a cabinet, a closet, the space below a raising floor, the space above a drop ceiling, the space in a hollow wall, an attic, a crawl space, the space between stored articles, the space between infrastructure support connections, a water carrying pipe, a shoe, a vacuum cleaner (modular attachment), a space in the head of a tooth brush, a space in the head of a brush, a space in headphones housing, a window frame, a water holding pond, a fridge for stored foods and drinks, a door frame.

40. A device according to claim 16 having in addition a computer system for controlling the output of the radiation unit.

41. A device according to claim 16 having at least one optical fiber for transferring real time spectroscopic data from the disinfecting regions the computer system.

42. A device according to claim 16 wherein the crystal is a non linear type of crystal capable of generating 2nd, and/or 3rd, and/or 4th harmonies of the primary frequency generated by the light source.

43. A device according to claims 16 wherein the primary frequency of the light source is in the IR region of the spectrum and at least one crystal is a capable of converting IR radiation frequency into the visible and/or UV radiation.

44. A device according to claim 16 wherein at least one fiber is connected to at least two elastic diffusive tips and wherein said tips are grouped together to form a brush reaching substantially into the liquids or gases to be disinfected.

45. A device according to claim 16 wherein walls of the conduit or chamber are transparent and layered having adequate refractive index profile for transmitting radiation.

46. A device according to claim 17 wherein the conduit or chamber is a algae reactor or biological reactor having industrial photosynthesis capability.

47. A device according to claim 17 wherein the conduit or chamber are a medical dialysis apparatus.

## Patentansprüche

1. Verfahren zur Ferndesinfektion von Flüssigkeiten und Gasen, umfassend:
Verteilen mindestens eines Lichtleiters in einem Bereich, der die zu desinfizierenden Flüssigkeiten oder Gase enthält,
Ausrichten mindestens einer Strahlungseinheit mit einer Lichtquelle hoher Intensität zu den Fasern, und
Bestrahlen der Flüssigkeiten oder Gase durch den Lichtleiter über einen vorbestimmten Zeitraum,
wobei die Strahlungseinheit ein Laser ist und die Primärstrahlungsfrequenz des Lasers vor oder im Desinfektionsbereich in eine andere Sekundärfrequenz umgewandelt wird, so dass die Frequenz der Sekundärstrahlung, die von den Lichtleitern abgegeben wird, von der Primärfrequenz des Lasers verschieden ist.

2. Verfahren nach Anspruch 1, wobei die Umwandlung der Primärfrequenz in die Sekundärfrequenz durch Frequenzvervielfachung erfolgt.

3. Verfahren nach Anspruch 1, wobei die Lichtleiter in den Körper oder die Wände eines Kanals oder einer Kammer, welche die zu desinfizierenden Flüssigkeiten oder Gase enthält, eingebracht sind.

4. Verfahren nach Anspruch 1, wobei mindestens ein seitlich emittierender Lichtleiter in dem Bereich angeordnet ist, der die zu desinfizierenden Flüssigkeiten oder Gase enthält.

5. Verfahren nach Anspruch 1, wobei mindestens ein Endankopplungs-Lichtleiter in dem Bereich angeordnet ist, der die zu desinfizierenden Flüssigkeiten oder Gase enthält.

6. Verfahren nach Anspruch 1, wobei ferner Spektroskopie-Daten in Echtzeit durch die Lichtleiter von dem Desinfektionsbereich zu einer beliebigen entfernten Stelle übermittelt werden zur Rückkopplungssteuerung des Desinfektionsverfahrens.

7. Verfahren nach Anspruch 1, wobei die Strahlungseinheit eine monochromatische Lichtquelle hoher Intensität mit einer einzelnen Wellenlänge besitzt.

8. Verfahren nach Anspruch 1, wobei die Strahlungseinheit eine polychromatische Lichtquelle hoher Intensität von etwa 249 bis 2400 nm ist.

9. Verfahren nach Anspruch 1, wobei die Strahlungseinheit eine gepulste Lichtquelle hoher Intensität ist.

10. Verfahren nach Anspruch 9, wobei die Pulse der Lichtquelle hoher Intensität gerichtet sind auf Lichtleiter, welche ausgewählt sind aus Unimode-, Multimode-, Gradientenindex-, Gradienten- oder die Polarisierung erhaltende Fasern oder Faserbündel.

11. Verfahren nach Anspruch 1, wobei die Strahlungseinheit eine stetige Lichtquelle hoher Intensität ist.

12. Verfahren nach Anspruch 1, wobei die Strahlungseinheit ein ultraviolettes Licht hoher Intensität von etwa 187 bis 400 nm besitzt.

13. Verfahren nach Anspruch 1, wobei die Strahlungseinheit ein Licht hoher Intensität im sichtbaren Bereich von etwa 400 bis 700 nm besitzt.

14. Verfahren nach Anspruch 1, wobei die Strahlungseinheit eine IR-Quelle hoher Intensität von etwa 800 bis 2400 nm besitzt.

15. Verfahren zur Ausstattung eines Netzwerks mit einer interaktiven optischen Infrastruktur zur Desinfektion von Flüssigkeiten oder Gasen, umfassend:
Verteilen in einem Kanal oder einer Kammer, welche die Flüssigkeit oder das Gas enthält, die oder das zu desinfizieren ist, mindestens einen Lichtleiter mit einem ersten Eingangs-Endanschluss und einem zweiten Ende, das sich im Wesentlichen bis in den Kanal oder die Kammer hineinerstreckt, und beim Eintritt in einem vorbestimmten Winkel mit mindestens einem modularen zweiten und/oder dritten und/oder vierten Frequenzvervielfachungskristall-Anschlussmodul verbunden ist, wobei der Kristall mit einer lichtdurchlässigen isolierenden Linse abschließt, die der zu desinfizierenden Flüssigkeit oder dem Gas zugewendet ist;
Ausrichten mindestens einer Strahlungseinheit mit einer Lichtquelle hoher Intensität in die Fasern;
Ankoppeln, direkt und/oder durch die Eingangs-Vakuumschnittstelle in dem ersten Endanschluss der Fasern, eines Lichts hoher Intensität mit einer Primärwellenlänge im sichtbaren und/oder NIR-und/oder IR- und/oder UV-Bereich des elektromagnetischen Spektrums;
Beleuchten oder Strahlen eines vorbestimmten Flüssigkeits- oder Gasvolumens über einen vorbestimmten Zeitraum mit dem gelieferten Licht mit einem Sekundärwellenlängenbereich im UVA- und/oder UVB- und/oder UVC- und/oder sichtbaren und/oder IR-Bereich oder einem Frequenzvielfachen oder einer umgewandelten Spektralkombination davon.

16. Vorrichtung zur Desinfektion von Flüssigkeiten und Gasen nach dem Verfahren aus Anspruch 1, umfassend einen Kanal oder eine Kammer, welche die zu desinfizierenden Flüssigkeiten oder Gase enthält, wobei mindestens ein Lichtleiter innerhalb oder um oder eingefügt in die Wände oder den Körper oder im Bereich um den Kanal oder die Kammer verteilt ist, und einen Laser mit einer Lichtquelle hoher Intensität, die in die Fasern gerichtet ist, wobei mindestens eine Kristallschnittstelle mit mindestens einem Endanschluss einer Lichtleitfaser oder einem Faserbündel verbunden oder darin integriert ist zur Umwandlung der Frequenz der Wellenlänge des ankommenden Primärpulses in eine niedrigere Wellenlänge des ausgehenden Pulses.

17. Vorrichtung nach Anspruch 16, wobei der Laser eine polychromatische oder monochromatische Lichtquelle von 220 bis etwa 2400 nm ist.

18. Vorrichtung nach Anspruch 16, wobei der Laser ausgewählt ist aus Blitzlicht, Faser-Laser, Festkörper-Laser, Gas-Laser und Kristall-Laser.

19. Vorrichtung nach Anspruch 16, wobei die Fasern in die Strahlungseinheit durch einen Vakuumflansch oder eine optische Kollimator-Schnittstelle gerichtet oder eine Schnittstelle bildend angeschlossen sind, was den Schadensschwellenwert der Fasern erhöht und das Zuführen beträchtlicher Lichtenergie einer bestimmten Spektralverteilung ermöglicht.

20. Vorrichtung nach Anspruch 16, besonders nützlich zum Stören des Brutzyklus von Schaben, wobei das von den Fasern ausgestrahlte Licht im sichtbaren Bereich des Spektrums liegt.

21. Vorrichtung nach Anspruch 17, wobei die Strahlungsquelle eine polychromatische Mikrowellen-Erregungs-lampe ist.

22. Vorrichtung nach Anspruch 17, wobei die Strahlungseinheit ersetzt ist durch einen Solarkollektor und/oder Strahlungsbündler, der das auf die Erde einfallende Sonnenlicht nutzbar macht.

23. Vorrichtung nach Anspruch 16 mit mindestens einem seitlich emittierenden Lichtleiter.

24. Vorrichtung nach Anspruch 16 mit mindestens einem Endankopplungs-Lichtleiter.

25. Vorrichtung nach Anspruch 16 mit Einrichtungen zum Halten der Fasern innerhalb des Kanals oder der Kammer.

26. Vorrichtung nach Anspruch 16 mit einer lichtdurchlässigen oder lichtundurchlässigen Hülse, welche die Fasern umhüllt.

27. Vorrichtung nach Anspruch 26, wobei die Hülse die Lichtleiter in einstückiger Weise umschließt.

28. Vorrichtung nach Anspruch 16, wobei die Lichtleiter in dem Kanal oder der Kammer räumlich verteilt sind, so dass in dem Kanal oder der Kammer mindestens ein Bereich konstruktiver Interferenz entsteht.

29. Vorrichtung nach Anspruch 16, wobei die Lichtleiter seitlich emittierende Fasern sind, die räumlich in einer Spirale oder Zickzack verteilt sind, so dass eine Ebene, ein Konus, ein Zylinder oder eine glatte Oberfläche oder eine Kombinationen davon entsteht.

30. Vorrichtung nach Anspruch 16, wobei die Faser entlang des eigenen Verlaufs zurückgebogen ist, so dass mindestens ein Bereich mit parallelem Faserverlauf entsteht.

31. Vorrichtung nach Anspruch 16 mit einem Reflektor-element, parallel zu mindestens einem Strahlungs-bereich der verteilten Fasern.

32. Vorrichtung nach Anspruch 16 mit einer reflektierenden Endkappe, die am hinteren Ende des Lichtleiters befestigt ist, so dass in dem Kanal, der Kammer oder einem anderen vorbestimmten Raum mindestens ein Bereich konstruktiver Interferenz entsteht.

33. Vorrichtung nach Anspruch 31, wobei die Faser eine seitlich emittierende Faser ist und das Reflektorteil ein integraler Bestandteil der Faser ist.

34. Vorrichtung nach Anspruch 17, wobei die Faser vom Typ Endankopplungs-Faser ist, und die Fasern der Größe nach angeordnet sind, so dass mindestens ein Bereich mit konstruktiver Interferenz entsteht.

35. Vorrichtung nach Anspruch 17, die zudem ein holographisches optisches Element aufweist, so dass in dem Kanal, der Kammer oder einem anderen vorbestimmten Raum mindestens ein Bereich konstruktiver Interferenz entsteht.

36. Vorrichtung nach Anspruch 16 mit einem ultravioletten Hologramm in dem Kanal oder der Kammer, wobei das Hologramm aus mindestens einer konstruktiven Interferenz gebildet ist und den Großteil des Innenraums des Kanals öder der Kammer einnimmt.

37. Vorrichtung nach Anspruch 17, wobei der Kanal oder die Kammer eine Filtriereinheit aufweist mit mindestens einem Porensieb, einer Membran, einer Oberflächenscheibe, Kapillaren, Magnetelementen oder Verbindungen zur Entfernung von Teilchenmaterial und die Lichtleiter auf oder in mindestens einen der Siebe, Membranen oder Oberflächenscheiben der Filtriereinheit integriert sind.

38. Vorrichtung nach Anspruch 16, wobei der Kanal oder die Kammer ein Abwasserrohr ist.

39. Vorrichtung nach Anspruch 16, wobei die Kammer ein geschlossener Raum ist, ausgewählt aus Belüftungs-Rauminhalten locker gepackter Erde, einer Kammer, einem Schrank, dem Raum unter einem Hebeboden, dem Raum über einer Falltür, der Raum in einer Hohlwand, ein Dachboden, ein Kriechraum, der Raum zwischen gelagerten Gegenständen, der Raum zwischen vernetzenden Stützverbindungen, eine Wasserleitung, ein Schuh, ein Staubsauger (Modulverbindung), der Raum im Zahnbürstenkopf, der Raum im Bürstenkopf, der Raum im Kopfhörergehäuse, ein Fensterrahmen, ein wassergefüllter Teich, ein Kühlschrank für gelagerte Lebensmittel und Getränke, ein Türrahmen.

40. Vorrichtung nach Anspruch 16, die zudem ein Computersystem zur Steuerung der Leistungsabgabe der Strahlungseinheit aufweist.

41. Vorrichtung nach Anspruch 16 mit mindestens einem Lichtleiter zur Echtzeitübertragung von Spektroskopiedaten aus den Desinfektionsbereichen an das Computersystem.

42. Vorrichtung nach Anspruch 16, wobei der Kristall vom nicht-linearen Kristalltyp ist, welcher fähig ist zweite, dritte und/oder vierte Oberwellen der Primärfrequenz, die durch die Lichtquelle gebildet werden, zu erzeugen.

43. Vorrichtung nach Anspruch 16, wobei die Primärfrequenz der Lichtquelle im IR-Bereich des Spektrums liegt und mindestens ein Kristall vermag, die IR-Strahlungsfrequenz in sichtbare und/oder UV-Strahlung umzuwandeln.

44. Vorrichtung nach Anspruch 16, wobei mindestens eine Faser mit mindestens zwei elastischen Diffusionsspitzen verbunden ist und die Spitzen zusammengefasst sind, so dass ein Pinsel entsteht, der im Wesentlichen in die zu desinfizierenden Flüssigkeiten oder Gase hineinreicht.

45. Vorrichtung nach Anspruch 16, wobei die Wände des Kanals oder der Kammer lichtdurchlässig und geschichtet sind mit einem angemessenen Brechungszahlprofil zur Strahlungsübertragung.

46. Vorrichtung nach Anspruch 17, wobei der Kanal oder die Kammer ein Algenreaktor oder ein biologischer Reaktor ist, der eine Photosyntheseleistung im Industriemaßstab aufweist.

47. Vorrichtung nach Anspruch 17, wobei der Kanal oder die Kammer medizinische Dialysevorrichtungen sind.

## Revendications

1. Procédé de désinfection à distance de liquides et de gaz comprenant la mise en place d'au moins une fibre optique dans la région contenant les liquides ou les gaz à désinfecter ; l'alignement d'au moins une unité de radiation ayant une source de lumière de haute intensité à l'intérieur desdites fibres et l'irradiation desdits liquides ou gaz par ladite fibre optique pendant une période de temps prédéterminée, dans lequel ladite unité de radiation est un laser et dans lequel la fréquence de radiation primaire du laser est convertie en une autre fréquence secondaire, avant le site de désinfection ou au niveau de celui-ci, de telle sorte que la radiation secondaire émise à partir des fibres optiques a une fréquence différente de la fréquence primaire du laser.

2. Procédé suivant la revendication 1, dans lequel la conversion de la fréquence primaire en une fréquence secondaire est générée harmoniquement.

3. Procédé suivant la revendication 1, dans lequel les fibres optiques sont intégrées à l'intérieur du corps ou des parois d'un conduit ou d'une chambre contenant les liquides ou les gaz à désinfecter.

4. Procédé suivant la revendication 1, ayant au moins une fibre optique émettant latéralement mise en place dans la région contenant les liquides ou les gaz à désinfecter.

5. Procédé suivant la revendication 1, ayant au moins une fibre optique à rayonnement terminal mise en place dans la région contenant les liquides ou les gaz à désinfecter.

6. Procédé suivant la revendication 1, transférant de plus en temps réel des données spectroscopiques au moyen de fibres optiques à partir de la région désinfectée vers un site éloigné quelconque pour la commande par rétroaction du traitement de désinfection.

7. Procédé suivant la revendication 1, dans lequel l'unité de radiation a une source de lumière de haute intensité monochromatique de longueur d'onde unique.

8. Procédé suivant la revendication 1, dans lequel l'unité de radiation est une source de lumière de haute intensité polychromatique d'environ 249 nm à environ 2400 nm.

9. Procédé suivant la revendication 1, dans lequel la source de radiation est une source de lumière pulsée de haute intensité.

10. Procédé suivant la revendication 9, dans lequel les impulsions provenant de la source de lumière de haute intensité sont alignées avec des fibres optiques choisies parmi des fibres monomodes, multimodes, à saut d'indice, à gradient d'indice, ou des fibres conservant la polarisation ou des paquets de fibres.

11. Procédé suivant la revendication 1, dans lequel l'unité de radiation est une source de lumière continue de haute intensité.

12. Procédé suivant la revendication 1, dans lequel l'unité de radiation a une lumière ultraviolette de haute intensité d'environ 187 nm à environ 400 nm.

13. Procédé suivant la revendication 1, dans lequel l'unité de radiation a une source de lumière visible de haute intensité d'environ 400 nm à environ 700 nm.

14. Procédé suivant la revendication 1, dans lequel l'unité de radiation a une source infrarouge de haute intensité d'environ 800 nm à environ 2400 nm.

15. Procédé pour équiper un réseau d'une infrastructure optique interactive pour la désinfection de liquides ou de gaz, comprenant : la mise en place dans un conduit ou une chambre contenant le liquide ou le gaz à désinfecter, d'au moins une fibre optique ayant un premier élément d'extrémité récepteur et une seconde extrémité se prolongeant de façon substantielle pour atteindre l'intérieur du conduit ou de la chambre, et couplée au niveau de l'entrée selon un angle prédéterminé, à au moins une interface d'un module à cristal de génération modulaire de 2ème et/ou 3ème et/ou 4ème harmoniques, dans lequel le cristal est terminé par une lentille transparente isolante faisant face aux liquides ou aux gaz à désinfecter ; l'alignement d'au moins une unité de radiation ayant une source de lumière de haute intensité à l'intérieur desdites fibres ; l'envoi directement et/ou à travers une interface réceptrice sous vide dans le premier élément d'extrémité desdites fibre d'une lumière de haute intensité ayant une longueur d'onde primaire dans les régions du visible et/ou de l'IR proche et/ou de l'IR et/ou de l'UV du spectre électromagnétique ; l'éclairage ou l'irradiation d'un volume prédéterminé de liquides ou de gaz avec la lumière fournie ayant une gamme de longueur d'onde secondaire dans les régions UVA et/ou UVB et/ou UVC et/ou visible et/ou IR ou une quelconque combinaison spectrale de celle-ci générée harmoniquement ou convertie pendant une période de temps prédéterminée.

16. Dispositif pour la désinfection de liquides et de gaz par le procédé suivant la revendication 1, comprenant un conduit ou une chambre contenant les liquides ou les gaz à désinfecter, au moins une fibre optique mise en place à l'intérieur ou autour ou intégrée à l'intérieur des parois ou du corps ou du voisinage dudit conduit ou de ladite chambre, et un laser ayant une source de lumière de haute intensité alignée à l'intérieur desdites fibres dans lequel au moins une interface de cristal est attachée ou intégrée à au moins un élément d'extrémité d'une fibre ou d'un paquet de fibres optiques pour convertir harmoniquement la longueur d'onde de l'impulsion primaire d'entrée en une longueur d'onde inférieure d'impulsion de sortie.

17. Dispositif suivant la revendication 16, dans lequel le laser est une source de lumière polychromatique ou monochromatique de 220 nm à environ 2400 nm.

18. Dispositif suivant la revendication 16, dans lequel le laser est choisi parmi une lampe flash, un laser à fibre, un laser à semiconducteurs, un laser à gaz, un laser à cristal.

19. Dispositif suivant la revendication 16, dans lequel les fibres sont alignées ou interfacées dans l'unité de radiation à travers une bride sous vide ou une interface optique d'alignement qui augmente le seuil de détérioration des fibres en permettant la distribution d'une énergie optique substantielle ayant une distribution spectrale spécifique.

20. Dispositif suivant la revendication 16, particulièrement utile pour perturber le cycle de reproduction des blattes dans lequel la lumière émise à partir des fibres est dans la région visible du spectre.

21. Dispositif suivant la revendication 17, dans lequel la source de radiation est une lampe à excitation micro-onde polychromatique.

22. Dispositif suivant la revendication 17, dans lequel l'unité de radiation est remplacée par un capteur solaire et/ou un concentrateur captant le rayonnement solaire global.

23. Dispositif suivant la revendication 16, ayant au moins une fibre optique émettant latéralement.

24. Dispositif suivant la revendication 16, ayant au moins une fibre optique à rayonnement terminal.

25. Dispositif suivant la revendication 16, ayant un moyen pour soutenir les fibres à l'intérieur du conduit ou de la chambre.

26. Dispositif suivant la revendication 16, ayant un manchon transparent ou opaque enfermant les fibres optiques.

27. Dispositif suivant la revendication 26, dans lequel le manchon renferme intégralement les fibres optiques.

28. Dispositif suivant la revendication 16, dans lequel les fibres dans le conduit ou la chambre sont disposées de façon géométrique pour la production d'au moins une région d'interférence constructive dans le conduit ou la chambre.

29. Dispositif suivant la revendication 16, dans lequel les fibres sont des fibres à émission latérale, disposées de façon géométrique en spirale ou en zigzag pour la formation d'un plan, d'un cône, d'un cylindre ou d'une surface lisse ou d'une combinaison de ceux-ci.

30. Dispositif suivant la revendication 16, dans lequel la fibre est repliée sur elle-même pour former au moins une section de chemin de fibre parallèle.

31. Dispositif suivant la revendication 16, ayant un élément réfléchissant parallèle à au moins une section émettant un rayonnement de la fibre mise en place.

32. Dispositif suivant la revendication 16, ayant une coupelle terminale réfléchissante fixée à l'extrémité terminale de la fibre optique pour la production d'au moins une région d'interférence constructive dans le conduit ou la chambre ou tout autre espace prédéterminé.

33. Dispositif suivant la revendication 31, dans lequel la fibre est une fibre à émission latérale et dans lequel l'élément réfléchissant est une partie intégrale de la fibre.

34. Dispositif suivant la revendication 17, dans lequel la fibre est un type de fibre à rayonnement terminal, et dans lequel les fibres sont disposées dimensionnellement pour la production d'au moins une région d'interférence constructive.

35. Dispositif suivant la revendication 17, ayant de plus un élément optique holographique pour la production d'au moins une région d'interférence constructive dans le conduit ou une chambre ou tout autre espace prédéterminé.

36. Dispositif suivant la revendication 16, ayant un hologramme ultraviolet dans le conduit ou la chambre et où ledit hologramme est formé à partir d'au moins une interférence constructive et ledit hologramme remplit la plus grande partie de l'espace intérieur du conduit ou de la chambre.

37. Dispositif suivant la revendication 17, dans lequel le conduit ou la chambre a une unité de filtration ayant au moins un tamis poreux ou une membrane, ou un disque superficiel ou des capillaires, ou des éléments magnétiques ou des composés pour l'élimination de matières particulaires et dans lequel les fibres optiques sont intégrées à l'intérieur ou sur au moins l'un des tamis ou membranes, ou disque de surface de l'unité de filtration.

38. Dispositif suivant la revendication 16, dans lequel le conduit ou la chambre est un tuyau d'égoûts.

39. Dispositif suivant la revendication 16, dans lequel la chambre est un espace clos choisi parmi les volumes d'aération de sol peu tassé, un cabinet, une petite pièce, l'espace situé en dessous d'une rampe, l'espace situé au dessus d'un plafond mansardé, l'espace situé dans une paroi creuse, les combles, un faux-plancher, l'espace situé entre des articles stockés, l'espace entre. des raccords de supports d'infrastructure, un tuyau transportant de l'eau, une chaussure, un aspirateur (attachement modulaire), un espace dans la tête d'une brosse à dents, un espace dans la tête d'une brosse, un espace dans des logements d'écouteur, un cadre de fenêtre, une pièce d'eau, un réfrigérateur pour le stockage d'aliments et de boissons, un chambranle de porte.

40. Dispositif suivant la revendication 16, ayant de plus un système d'ordinateur pour la commande de la sortie de l'unité de radiation.

41. Dispositif suivant la revendication 16, ayant au moins une fibre optique pour transférer des données spectroscopiques en temps réel provenant des régions de désinfection vers l'ordinateur.

42. Dispositif suivant la revendication 16, dans lequel le cristal est un type de cristal non linéaire capable de générer les 2ème et/ou 3ème et/ou 4ème harmoniques de la fréquence primaire générée par la source de lumière.

43. Dispositif suivant la revendication 16, dans lequel la fréquence primaire de la source de lumière est dans la région IR du spectre et où au moins un cristal est capable de convertir une fréquence de radiation IR en radiation visible et/ou UV.

44. Dispositif suivant la revendication 16, dans lequel au moins une fibre est raccordée à au moins deux extrémités de diffusion élastiques et dans lequel lesdites extrémités sont groupées ensemble pour former une brosse qui pénètre substantiellement à l'intérieur des liquides ou des gaz à désinfecter.

45. Dispositif suivant la revendication 16, dans lequel les parois du conduit ou de la chambre sont transparentes et formées de couches ayant un profil d'indice de réfraction adéquat pour la transmission du rayonnement.

46. Dispositif suivant la revendication 17, dans lequel le conduit ou la chambre est un réacteur à algues ou un réacteur biologique capable de réaliser une photosynthèse industrielle.

47. Dispositif suivant la revendication 17, dans lequel le conduit ou la chambre est un appareil de dialyse médical.
